# EUROPEAN PATENT APPLICATION

(11) **EP 4 851 156 A1**
(43) Date of publication of application: **09.12.2026**
(21) Application number: 25747785.1
(22) Date of filing: 24.01.2025
(51) Int. Cl.: C07D 409/12, C07D 333/02, C07D 333/00, C07D 417/02, C07D 417/14, A61K 31/381, A61P 29/00

(54) **SULFUR-CONTAINING HETEROCYCLIC DERIVATIVE AND USE THEREOF IN MEDICINE**

(30) Priority: 01.02.2024 CN 202410143011; 25.04.2024 CN 202410502462; 08.07.2024 CN 202410905045; 20.09.2024 CN 202411316127; 06.11.2024 CN 202411575239
(71) Applicant: Haisco Pharmaceutical Group Co., Ltd., Shannan, Xizang 856099 (CN)
(72) Inventor: ZHANG, Chen, Chengdu, Sichuan 611130 (CN); LEI, Ming, Chengdu, Sichuan 611130 (CN); WENG, Guanglin, Chengdu, Sichuan 611130 (CN); GOU, Xiaoli, Chengdu, Sichuan 611130 (CN); MENG, Qingyuan, Chengdu, Sichuan 611130 (CN); LI, Yao, Chengdu, Sichuan 611130 (CN); YAN, Pangke, Chengdu, Sichuan 611130 (CN)
(74) Representative: Hoffmann Eitle
(86) International application number: PCT/CN2025/074548
(87) International publication number: WO 2025/162217

(57) **Abstract**

The present invention relates to a compound of general formula (I) or a stereoisomer, a deuterate, a solvate, a prodrug, a metabolite, a pharmaceutically acceptable salt, or a cocrystal thereof, and intermediates thereof, preparation methods therefor, and use thereof in the preparation of a medicament for treating pain.

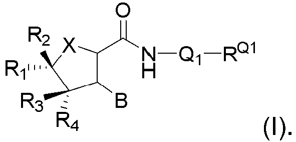

## Description

### TECHNICAL FIELD

The present invention relates to a compound of general formula (I) or a stereoisomer, a deuterate, a solvate, a prodrug, a metabolite, a pharmaceutically acceptable salt, or a cocrystal thereof, and intermediates thereof, preparation methods therefor, and use thereof in the preparation of a medicament for treating or alleviating pain.

### BACKGROUND ART

Pain originates from nociceptors of the peripheral nervous system. The nociceptors can convert a sensed thermal, mechanical, or chemical stimulus into nerve impulses (action potential) and transmit the same via afferent nerve fibers to the cell body portions thereof located in the dorsal root ganglia (DRG), and finally to a higher nerve center, causing pain sensation. On the other hand, the generation and transmission of action potentials in neurons are dependent on voltage-gated sodium channels (VGSCs) on cell membranes. When the cell membranes are depolarized, the sodium channels are activated and opened, causing an influx of sodium ions that further depolarizes the cell membranes, resulting in the generation of action potentials.

A VGSC consists of a pore-forming α-subunit (about 260 kDa) and an associated smaller-sized (30 to 40 kDa) β-subunit. The related α-subunit family consists of 10 members, 9 of which (Nav1.1 to 1.9) are voltage-gated. Nav1.8 is encoded by the gene SCN10A and is preferentially expressed in peripheral sensory neurons. It has been shown that the action potentials in these neurons can be modified. Presently, Nav1.8 transcripts and proteins have been discovered in dorsal root ganglion (DRG) neurons. Nav1.8 has not been detected in non-neuronal tissues (such as heart and skeletal muscle) or the central nervous system (including the brain and spinal cord).

The key role of Nav1.8 in pain signaling has been supported by a plethora of evidence. Based on a series of animal experiments as well as human genetic evidence, selective inhibition of Nav1.8 has the potential to become a novel analgesic therapy. Currently, some drugs targeting this target have entered clinical research.

### SUMMARY OF THE INVENTION

An object of the present invention is to provide a sulfur-containing heterocyclic derivative having an inhibitory activity against Nav1.8. The compound selectively inhibits Nav1.8, and can effectively reduce side effects. The compound has good analgesic activity, liver microsome stability, pharmacokinetic properties, and oral bioavailability.

The present invention relates to a compound of general formula (I) or a stereoisomer, a deuterate, a solvate, a prodrug, a metabolite, a pharmaceutically acceptable salt, or a cocrystal thereof, 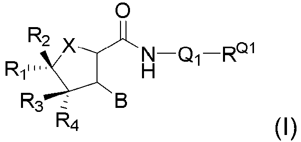
in some embodiments, the compound of general formula (I) is selected from general formulas (la), (Ib), (Ic), or (Id): 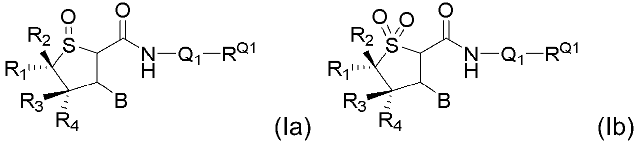 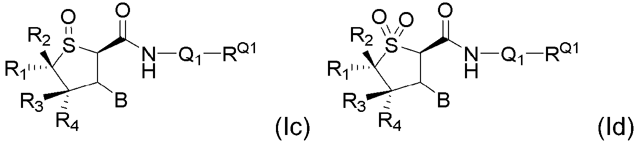
in some embodiments, the compound of general formula (I) is selected from general formulas (Ic-1) or (Id-1) 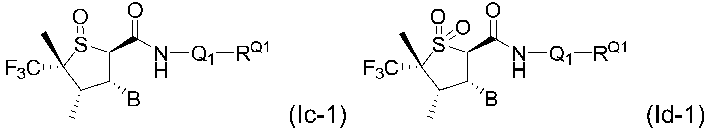
in some embodiments, X is selected from -S(O)- or -S(O)₂;
in some embodiments, Q₁ is selected from C₆₋₁₀ aryl, 5- to 10-membered heteroaryl, C₅₋₁₀ carbocycle, 5- to 10-membered heterocycle, and 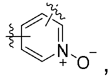 the aryl, heteroaryl, carbocycle, or heterocycle being optionally substituted with 1 to 5 R^{q}'s;
in some embodiments, Q₁ is selected from phenyl, benzo C₄₋₆ carbocycle, benzo 4- to 6-membered heterocycle, 5- to 6-membered heteroaryl, 5- to 6-membered heterocycle, 8- to 10-membered fused heteroaryl, or 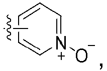 Q₁ being optionally substituted with 1 to 4 R^{q}'s;
in some embodiments, Q ₁ is selected from 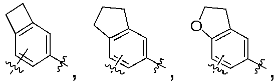 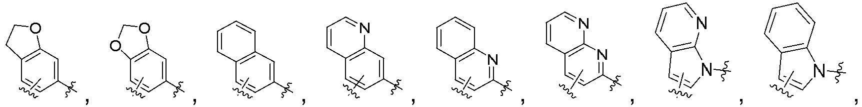 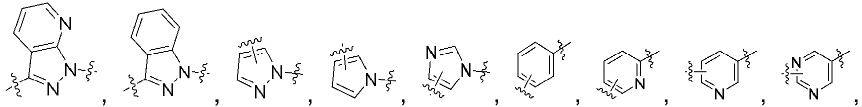 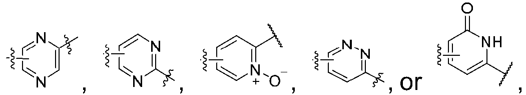 Q₁ being optionally substituted with 1 to 4 R^{q}'s;
in some embodiments, 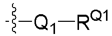 is selected from 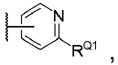 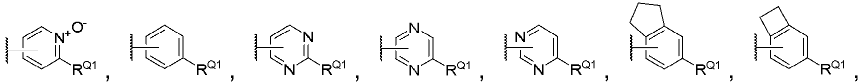 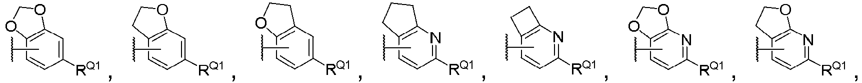 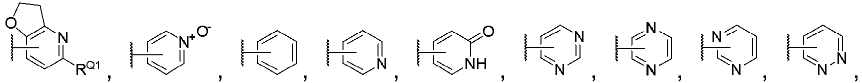 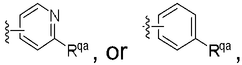 Q₁ being optionally substituted with 1 to 3 R^{q}'s;
in some embodiments, R^{qa} is selected from 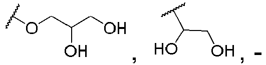 CH₂OH, -CF₂CH₂OH, NH₂, -P(=O)(CH₃)₂, -P(=O)(CH₂CH₃)₂, or - P(=O)(CH₃)(cyclopropyl);
in some embodiments, 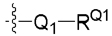 is selected from 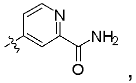 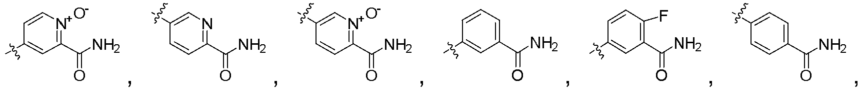 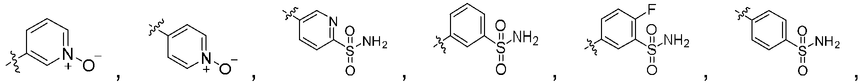 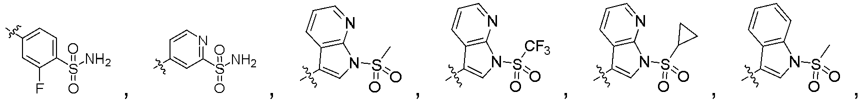 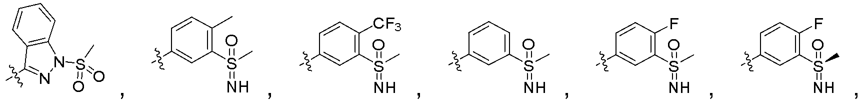 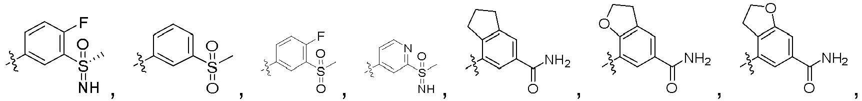 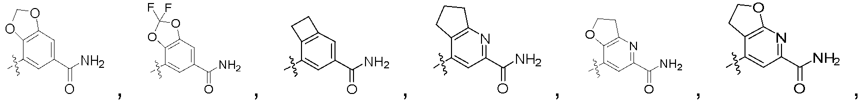 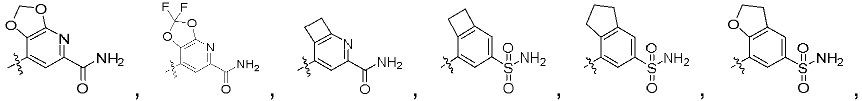 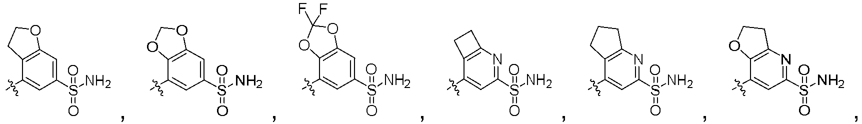 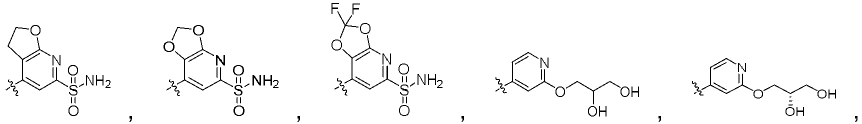 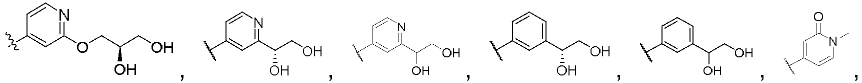 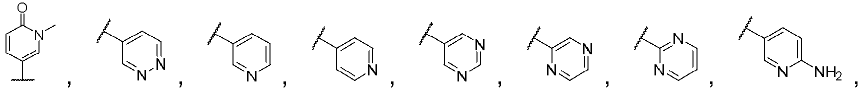 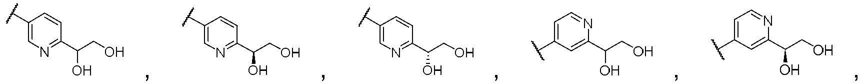 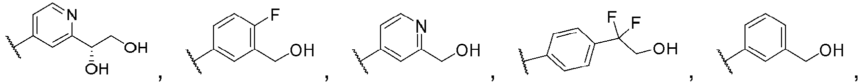 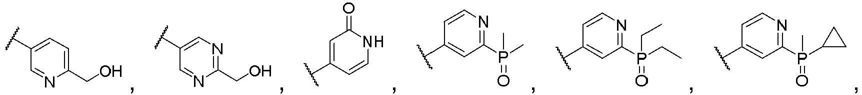 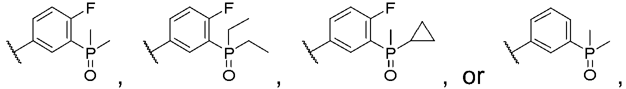 Q₁ being optionally substituted with 1 to 3 R^{q}'s; in some embodiments, R^{Q1} is selected from H, NR^{q1}R^{q2}, -C(=O)NR^{q1}R^{q2}, -S(=O)₂NR^{q1}R^{q2}, OH, =O, -OR^{q1}, -C(=O)R^{q1}, - S(=O)₂R^{q1}, -S(=O)(=NR^{q1})R^{q2}, or -P(=O)R^{q1}R^{q2};
in some embodiments, R^{Q1} is selected from -C(=O)NR^{q1}R^{q2} or - S(=O)₂NR^{q1}R^{q2};
in some embodiments, R^{q1} and R^{q2} are each independently selected from H, C₁₋₆ alkyl, C₃₋₆ carbocycle, or 4- to 7-membered heterocycle, the alkyl, carbocycle, or heterocycle being optionally substituted with 1 to 4 R^{k}'s;
in some embodiments, R^{q1} and R^{q2} are each independently selected from H, C₁₋₄ alkyl, C₃₋₆ carbocycle, or 4- to 7-membered heterocycle, the alkyl, carbocycle, or heterocycle being optionally substituted with 1 to 4 R^{k}'s;
in some embodiments, R^{q1} and R^{q2} are each independently selected from H, methyl, ethyl, propyl, isopropyl, cyclopropyl, cyclobutyl, or cyclopentyl, the methyl, ethyl, propyl, isopropyl, cyclopropyl, cyclobutyl, or cyclopentyl being optionally substituted with 1 to 4 R^{k}'s;
in some embodiments, alternatively, R^{q1} and R^{q2} are directly connected to form a 4- to 7-membered heterocycle, the heterocycle being optionally substituted with 1 to 4 R^{k}'s;
in some embodiments, B is selected from C₆₋₁₀ aryl, 5- to 10-membered heteroaryl, C₅₋₁₀ carbocycle, or 5- to 10-membered heterocycle, the aryl, heteroaryl, carbocycle, or heterocycle being optionally substituted with 1 to 5 R^{B}'s;
in some embodiments, B is selected from phenyl, benzo C₄₋₆ carbocycle, benzo 4- to 6-membered heterocycle, 5- to 6-membered heteroaryl, or 8- to 10-membered fused heteroaryl, B being optionally substituted with 1 to 4 R^{B}'s;
in some embodiments, B is selected from 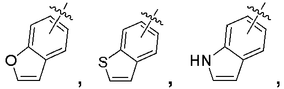 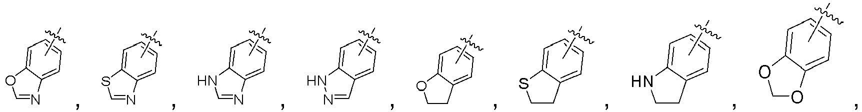 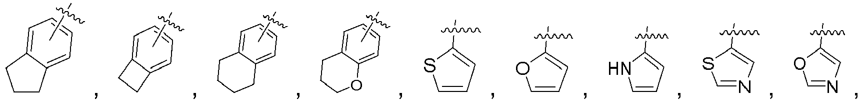 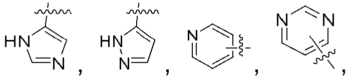 or phenyl, B being optionally substituted with 1 to 4 R^{B}'s;
in some embodiments, B is selected from 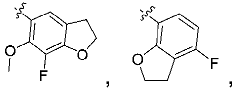 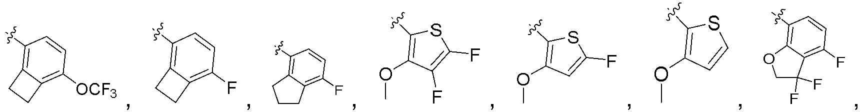 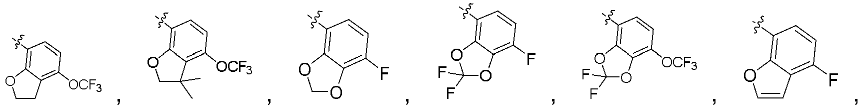 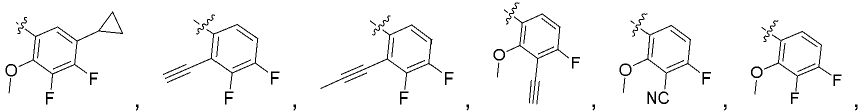 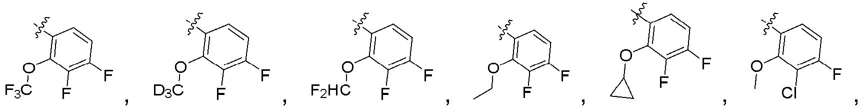 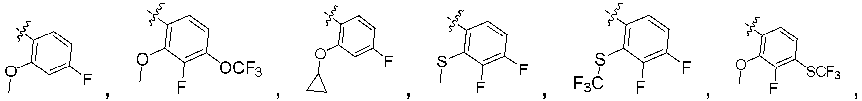 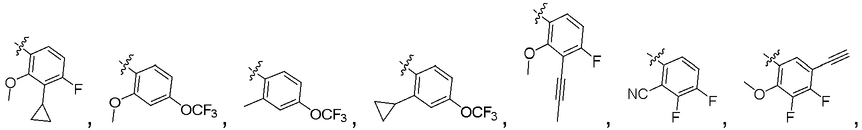 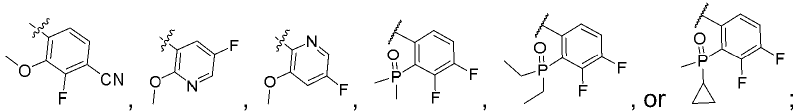
in some embodiments, B is selected from 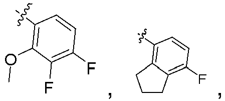 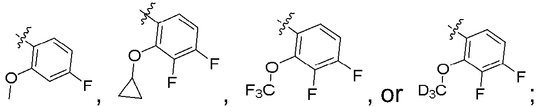
in some embodiments, R¹, R², R³, and R⁴ are each independently selected from H, halogen, CN, OH, C₁₋₆ alkyl, C₂₋₆ alkenyl, C₂₋₆ alkynyl, -OC₁₋₆ alkyl, -SC₁₋₆ alkyl, C₃₋₆ carbocycle, or 3- to 7-membered heterocycle, the alkyl, alkenyl, alkynyl, carbocycle, or heterocycle being optionally substituted with 1 to 4 R^{k}'s;
in some embodiments, R¹, R², R³, and R⁴ are each independently selected from H, halogen, CN, OH, C₁₋₄ alkyl, C₂₋₄ alkenyl, C₂₋₄ alkynyl, -OC₁₋₄ alkyl, -SC₁₋₄ alkyl, C₃₋₆ carbocycle, or 3- to 7-membered heterocycle, the alkyl, alkenyl, alkynyl, carbocycle, or heterocycle being optionally substituted with 1 to 4 R^{k}'s;
in some embodiments, R¹, R², R³, and R⁴ are each independently selected from H, F, Cl, Br, cyano, methyl, ethyl, methoxy, ethoxy, methylthio, cyclopropyl, cyclobutyl, ethenyl, or ethynyl, the methyl, ethyl, methoxy, ethoxy, methylthio, cyclopropyl, cyclobutyl, ethenyl, or ethynyl being optionally substituted with 1 to 4 R^{k}'s;
in some embodiments, R¹, R², R³, and R⁴ are each independently selected from H, methyl, ethyl, CH₂F, CHF₂, or CF₃;
in some embodiments, R¹ and R⁴ are directly connected to form a C₃₋₆ carbocycle or 4- to 7-membered heterocycle, the carbocycle or heterocycle being optionally substituted with 1 to 6 R^{k}'s;
in some embodiments, R¹ and R⁴ are directly connected to form a C₃₋₆ carbocycle, the carbocycle being optionally substituted with 1 to 4 R^{k}'s;
in some embodiments, R^{q} and R^{B} are each independently selected from H, deuterium, =O, halogen, CN, OH, NH₂, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, -SC₁₋₆ alkyl, C₃₋₇ carbocycle, - OC₃₋₇ carbocycle, 3- to 7-membered heterocycle, or -P(=O)R^{q1}R^{q2}, the alkyl, alkenyl, alkynyl, carbocycle, or heterocycle being optionally substituted with 1 to 4 R^{k}'s;
in some embodiments, R^{q} and R^{B} are each independently selected from H, deuterium, =O, halogen, CN, OH, NH₂, NHC₁₋₄ alkyl, N(C₁₋₄ alkyl)₂, C₁₋₄ alkyl, C₂₋₄ alkenyl, C₂₋₄ alkynyl, -OC₁₋₄ alkyl, -SC₁₋₄ alkyl, C₃₋₇ carbocycle, - OC₃₋₇ carbocycle, 3- to 7-membered heterocycle, or -P(=O)R^{q1}R^{q2}, the alkyl, alkenyl, alkynyl, carbocycle, or heterocycle being optionally substituted with 1 to 4 R^{k}'s;
in some embodiments, R^{q} and R^{B} are each independently selected from H, deuterium, =O, F, Cl, Br, cyano, methyl, ethyl, isopropyl, methoxy, ethoxy, isopropyloxy, methylthio, cyclopropyl, cyclopropyl-O-, cyclobutyl, ethenyl, ethynyl, -P(=O)(CH₃)₂, -P(=O)(CH₂CH₃)₂, or - P(=O)(CH₃)(cyclopropyl), the methyl, ethyl, isopropyl, methoxy, ethoxy, isopropyloxy, methylthio, cyclopropyl, cyclobutyl, ethenyl, or ethynyl being optionally substituted with 1 to 4 R^{k}'s;
in some embodiments, R^{q} and R^{B} are each independently selected from H, =O, F, CI, Br, cyano, CH₂F, CHF₂, CF₃, -OCH₂F, -OCHF₂, -OCF₃, - OCD₃, methyl, -S-methyl, -S-CF₃, ethyl, isopropyl, ethynyl, methoxy, ethoxy, isopropyloxy, propyloxy, cyclopropyl, -O-cyclopropyl, -P(=O)(CH₃)₂, - P(=O)(CH₂CH₃)₂, or -P(=O)(CH₃)(cyclopropyl), the methyl, ethyl, isopropyl, ethynyl, methoxy, ethoxy, isopropyloxy, propyloxy, or cyclopropyl being optionally substituted with 1 to 4 R^{k}'s;
in some embodiments, R^{k} in each occurrence is independently selected from deuterium, =O, halogen, CN, OH, NH₂, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, -SC₁₋₆ alkyl, -O-C₃₋₆ carbocycle, -O-3- to 7-membered heterocycle, -NH-C₃₋₆ carbocycle, -NH-3- to 7-membered heterocycle, -C₁₋₄ alkylene-C₃₋₆ carbocycle, -C₁₋₄ alkylene-3- to 7-membered heterocycle, C₃₋₆ carbocycle, or 3- to 7-membered heterocycle, the alkyl, alkylene, alkenyl, alkynyl, carbocycle, or heterocycle being optionally substituted with 1 to 4 substituents selected from deuterium, halogen, =O, CN, OH, NH₂, C₁₋₆ alkyl, or C₁₋₆ alkoxy;
in some embodiments, R^{k} in each occurrence is independently selected from deuterium, =O, halogen, CN, OH, NH₂, NHC₁₋₄ alkyl, N(C₁₋₄ alkyl)₂, C₁₋₄ alkyl, C₂₋₄ alkenyl, C₂₋₄ alkynyl, -OC₁₋₄ alkyl, -SC₁₋₄ alkyl, -O-C₃₋₆ carbocycle, or -O-3- to 7-membered heterocycle, the alkyl, alkenyl, alkynyl, carbocycle, or heterocycle being optionally substituted with 1 to 4 substituents selected from deuterium, halogen, CN, OH, NH₂, C₁₋₄ alkyl, or C₁₋₄ alkoxy;
in some embodiments, R^{k} in each occurrence is independently selected from deuterium, =O, F, CI, Br, I, CN, OH, NH₂, NH(CH₃), NH(CH₂CH₃), N(CH₃)₂, N(CH₂CH₃)₂, methyl, ethyl, ethenyl, ethynyl, methoxy, ethoxy, methylthio, -O-cyclopropyl, -NH-cyclopropyl, -CH₂-cyclopropyl, -CH₂-cyclobutyl, -CH₂-cyclopentyl, -CH₂-cyclohexyl, cyclopropyl, cyclobutyl, cyclopentyl, or cyclohexyl, the methyl, ethyl, ethenyl, ethynyl, methoxy, ethoxy, methylthio, cyclopropyl, cyclobutyl, cyclopentyl, or cyclohexyl being optionally substituted with 1 to 4 substituents selected from deuterium, halogen, CN, OH, NH₂, C₁₋₄ alkyl, or C₁₋₄ alkoxy; and
in some embodiments, R^{k} in each occurrence is independently selected from deuterium, F, CI, Br, I, CN, OH, -CH₂OH, methyl, ethyl, ethenyl, ethynyl, methoxy, ethoxy, methylthio, -O-cyclopropyl, -NH-cyclopropyl, -CH₂-cyclopropyl, -CH₂-cyclobutyl, -CH₂-cyclopentyl, -CH₂-cyclohexyl, cyclopropyl, cyclobutyl, cyclopentyl, or cyclohexyl.

As a first embodiment of the present invention, a compound of the following general formula (I) or a stereoisomer, a deuterate, a solvate, a prodrug, a metabolite, a pharmaceutically acceptable salt, or a cocrystal thereof, 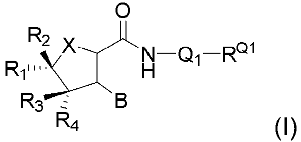
X is selected from -S(O)- or -S(O)₂;
Q₁ is selected from C₆₋₁₀ aryl, 5- to 10-membered heteroaryl, C₅₋₁₀ carbocycle, 5- to 10-membered heterocycle, or 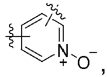 the aryl, heteroaryl, carbocycle, or heterocycle being optionally substituted with 1 to 5 R^{q}'s;
R^{Q1} is selected from H, NR^{q1}R^{q2}, -C(=O)NR^{q1}R^{q2}, -S(=O)₂NR^{q1}R^{q2}, OH, =O, -OR^{q1}, -C(=O)R^{q1}, -S(=O)₂R^{q1}, -S(=O)(=NR^{q1})R^{q2}, or -P(=O)R^{q1}R^{q2};
R^{q1} and R^{q2} are each independently selected from H, C₁₋₆ alkyl, C₃₋₆ carbocycle, or 4- to 7-membered heterocycle, the alkyl, carbocycle, or heterocycle being optionally substituted with 1 to 4 R^{k}'s;
alternatively, R^{q1} and R^{q2} are directly connected to form a 4- to 7-membered heterocycle, the heterocycle being optionally substituted with 1 to 4 R^{k}'s;
B is selected from C₆₋₁₀ aryl, 5- to 10-membered heteroaryl, C₅₋₁₀ carbocycle, or 5- to 10-membered heterocycle, the aryl, heteroaryl, carbocycle, or heterocycle being optionally substituted with 1 to 5 R^{B}'s;
R¹, R², R³, and R⁴ are each independently selected from H, halogen, CN, OH, C₁₋₆ alkyl, C₂₋₆ alkenyl, C₂₋₆ alkynyl, -OC₁₋₆ alkyl, -SC₁₋₆ alkyl, C₃₋₆ carbocycle, or 3- to 7-membered heterocycle, the alkyl, alkenyl, alkynyl, carbocycle, or heterocycle being optionally substituted with 1 to 4 R^{k}'s;
alternatively, R¹ and R⁴ are directly connected to form a C₃₋₆ carbocycle or 4- to 7-membered heterocycle, the carbocycle or heterocycle being optionally substituted with 1 to 6 R^{k}'s;
R^{q} and R^{B} are each independently selected from H, deuterium, =O, halogen, CN, OH, NH₂, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, -SC₁₋₆ alkyl, C₃₋₇ carbocycle, -OC₃₋₇ carbocycle, 3- to 7-membered heterocycle, or -P(=O)R^{q1}R^{q2}, the alkyl, alkenyl, alkynyl, carbocycle, or heterocycle being optionally substituted with 1 to 4 R^{k}'s; and
R^{k} in each occurrence is independently selected from deuterium, =O, halogen, CN, OH, NH₂, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, -SC₁₋₆ alkyl, -O-C₃₋₆ carbocycle, -O-3- to 7-membered heterocycle, -NH-C₃₋₆ carbocycle, -NH-3- to 7-membered heterocycle, -C₁₋₄ alkylene-C₃₋₆ carbocycle, -C₁₋₄ alkylene-3- to 7-membered heterocycle, C₃₋₆ carbocycle, or 3- to 7-membered heterocycle, the alkyl, alkylene, alkenyl, alkynyl, carbocycle, or heterocycle being optionally substituted with 1 to 4 substituents selected from deuterium, halogen, =O, CN, OH, NH₂, C₁₋₆ alkyl, or C₁₋₆ alkoxy.

As a second embodiment of the present invention, a compound of the above general formula (I) or a stereoisomer, a deuterate, a solvate, a prodrug, a metabolite, a pharmaceutically acceptable salt, or a cocrystal thereof, wherein
R^{q1} and R^{q2} are each independently selected from H, C₁₋₄ alkyl, C₃₋₆ carbocycle, or 4- to 7-membered heterocycle, the alkyl, carbocycle, or heterocycle being optionally substituted with 1 to 4 R^{k}'s;
alternatively, R^{q1} and R^{q2} are directly connected to form a 4- to 7-membered heterocycle, the heterocycle being optionally substituted with 1 to 4 R^{k}'s;
Q₁ is selected from phenyl, benzo C₄₋₆ carbocycle, benzo 4- to 6-membered heterocycle, 5- to 6-membered heteroaryl, 5- to 6-membered heterocycle, 8- to 10-membered fused heteroaryl, or 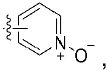 Q₁ being optionally substituted with 1 to 4 R^{q}'s;
B is selected from phenyl, benzo C₄₋₆ carbocycle, benzo 4- to 6-membered heterocycle, 5- to 6-membered heteroaryl, or 8- to 10-membered fused heteroaryl, B being optionally substituted with 1 to 4 R^{B}'s;
R¹, R², R³, and R⁴ are each independently selected from H, halogen, CN, OH, C₁₋₄ alkyl, C₂₋₄ alkenyl, C₂₋₄ alkynyl, -OC₁₋₄ alkyl, -SC₁₋₄ alkyl, C₃₋₆ carbocycle, or 3- to 7-membered heterocycle, the alkyl, alkenyl, alkynyl, carbocycle, or heterocycle being optionally substituted with 1 to 4 R^{k}'s;
alternatively, R¹ and R⁴ are directly connected to form a C₃₋₆ carbocycle or 4- to 7-membered heterocycle, the carbocycle or heterocycle being optionally substituted with 1 to 4 R^{k}'s;
R^{q} and R^{B} are each independently selected from H, deuterium, =O, halogen, CN, OH, NH₂, NHC₁₋₄ alkyl, N(C₁₋₄ alkyl)₂, C₁₋₄ alkyl, C₂₋₄ alkenyl, C₂₋₄ alkynyl, -OC₁₋₄ alkyl, -SC₁₋₄ alkyl, C₃₋₇ carbocycle, -OC₃₋₇ carbocycle, 3- to 7-membered heterocycle, or -P(=O)R^{q1}R^{q2}, the alkyl, alkenyl, alkynyl, carbocycle, or heterocycle being optionally substituted with 1 to 4 R^{k}'s; and
R^{k} in each occurrence is independently selected from deuterium, =O, halogen, CN, OH, NH₂, NHC₁₋₄ alkyl, N(C₁₋₄ alkyl)₂, C₁₋₄ alkyl, C₂₋₄ alkenyl, C₂₋₄ alkynyl, -OC₁₋₄ alkyl, -SC₁₋₄ alkyl, -O-C₃₋₆ carbocycle, or -O-3- to 7-membered heterocycle, the alkyl, alkenyl, alkynyl, carbocycle, or heterocycle being optionally substituted with 1 to 4 substituents selected from deuterium, halogen, CN, OH, NH₂, C₁₋₄ alkyl, or C₁₋₄ alkoxy; and
the other groups are as defined in the first embodiment of the present invention.

As a third embodiment of the present invention, a compound of the above general formula (I) or a stereoisomer, a deuterate, a solvate, a prodrug, a metabolite, a pharmaceutically acceptable salt, or a cocrystal thereof, wherein
R^{q1} and R^{q2} are each independently selected from H, methyl, ethyl, propyl, isopropyl, cyclopropyl, cyclobutyl, or cyclopentyl, the methyl, ethyl, propyl, isopropyl, cyclopropyl, cyclobutyl, or cyclopentyl being optionally substituted with 1 to 4 R^{k}'s;
R¹, R², R³, and R⁴ are each independently selected from H, F, Cl, Br, cyano, methyl, ethyl, methoxy, ethoxy, methylthio, cyclopropyl, cyclobutyl, ethenyl, or ethynyl, the methyl, ethyl, methoxy, ethoxy, methylthio, cyclopropyl, cyclobutyl, ethenyl, or ethynyl being optionally substituted with 1 to 4 R^{k}'s;
alternatively, R¹ and R⁴ are directly connected to form a C₃₋₆ carbocycle, the carbocycle being optionally substituted with 1 to 4 R^{k}'s;
B is selected from 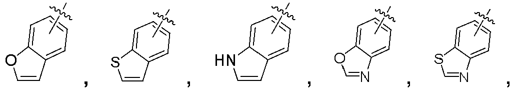 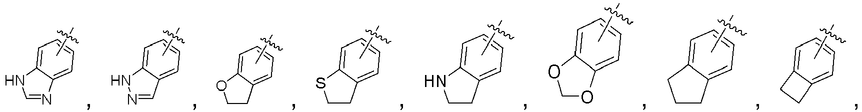 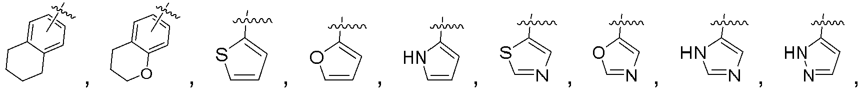 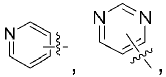 or phenyl, B being optionally substituted with 1 to 4 R^{B}'s;
R^{q} and R^{B} are each independently selected from H, deuterium, =O, F, Cl, Br, cyano, methyl, ethyl, isopropyl, methoxy, ethoxy, isopropyloxy, methylthio, cyclopropyl, cyclopropyl-O-, cyclobutyl, ethenyl, ethynyl, - P(=O)(CH₃)₂, -P(=O)(CH₂CH₃)₂, or -P(=O)(CH₃)(cyclopropyl), the methyl, ethyl, isopropyl, methoxy, ethoxy, isopropyloxy, methylthio, cyclopropyl, cyclobutyl, ethenyl, or ethynyl being optionally substituted with 1 to 4 R^{k}'s;
R^{k} in each occurrence is independently selected from deuterium, =O, F, CI, Br, I, CN, OH, NH₂, NH(CH₃), NH(CH₂CH₃), N(CH₃)₂, N(CH₂CH₃)₂, methyl, ethyl, ethenyl, ethynyl, methoxy, ethoxy, methylthio, -O-cyclopropyl, - NH-cyclopropyl, -CH₂-cyclopropyl, -CH₂-cyclobutyl, -CH₂-cyclopentyl, -CH₂-cyclohexyl, cyclopropyl, cyclobutyl, cyclopentyl, or cyclohexyl, the methyl, ethyl, ethenyl, ethynyl, methoxy, ethoxy, methylthio, cyclopropyl, cyclobutyl, cyclopentyl, or cyclohexyl being optionally substituted with 1 to 4 substituents selected from deuterium, halogen, CN, OH, NH₂, C₁₋₄ alkyl, or C₁₋₄ alkoxy; and
the other groups are as defined in the first or second embodiment of the present invention.

As a fourth embodiment of the present invention, a compound of the above general formula (I) or a stereoisomer, a deuterate, a solvate, a prodrug, a metabolite, a pharmaceutically acceptable salt, or a cocrystal thereof, wherein
Q₁ is selected from 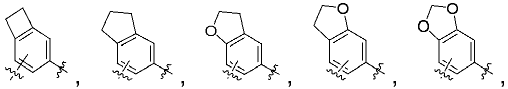 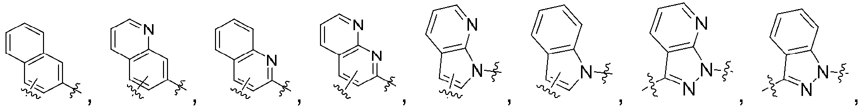 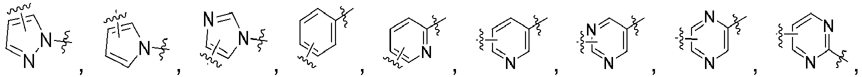 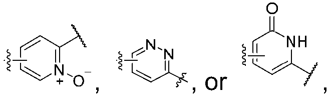 Q₁ being optionally substituted with 1 to 4 R^{q}'s;
R¹, R², R³, and R⁴ are each independently selected from H, methyl, ethyl, CH₂F, CHF₂, or CF₃;
R^{q} and R^{B} are each independently selected from H, =O, F, Cl, Br, cyano, CH₂F, CHF₂, CF₃, -OCH₂F, -OCHF₂, -OCF₃, -OCD₃, methyl, -S-methyl, -S-CF₃, ethyl, isopropyl, ethynyl, methoxy, ethoxy, isopropyloxy, propyloxy, cyclopropyl, -O-cyclopropyl, -P(=O)(CH₃)₂, -P(=O)(CH₂CH₃)₂, or - P(=O)(CH₃)(cyclopropyl), the methyl, ethyl, isopropyl, ethynyl, methoxy, ethoxy, isopropyloxy, propyloxy, or cyclopropyl being optionally substituted with 1 to 4 R^{k}'s;
R^{k} in each occurrence is independently selected from deuterium, F, Cl, Br, I, CN, OH, -CH₂OH, methyl, ethyl, ethenyl, ethynyl, methoxy, ethoxy, methylthio, -O-cyclopropyl, -NH-cyclopropyl, -CH₂-cyclopropyl, -CH₂-cyclobutyl, -CH₂-cyclopentyl, -CH₂-cyclohexyl, cyclopropyl, cyclobutyl, cyclopentyl, or cyclohexyl; and
the other groups are as defined in the first, second, or third embodiment of the present invention.

As a fifth embodiment of the present invention, a compound of the above general formula (I) or a stereoisomer, a deuterate, a solvate, a prodrug, a metabolite, a pharmaceutically acceptable salt, or a cocrystal thereof, wherein 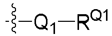 is selected from 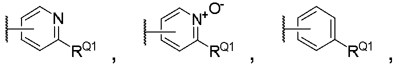 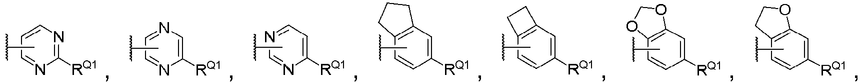 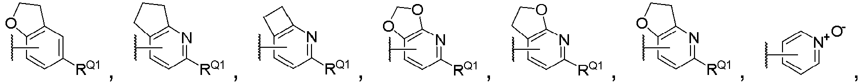 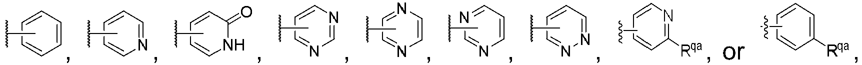 Q₁ being optionally substituted with 1 to 3 R^{q}'s;
R^{qa} is selected from 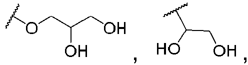 -CH₂OH, -CF₂CH₂OH, NH₂, -P(=O)(CH₃)₂, -P(=O)(CH₂CH₃)₂, or -P(=O)(CH₃)(cyclopropyl); and
the other groups are as defined in the first, second, third, or fourth embodiment of the present invention.

As a sixth embodiment of the present invention, a compound of the above general formula (I) or a stereoisomer, a deuterate, a solvate, a prodrug, a metabolite, a pharmaceutically acceptable salt, or a cocrystal thereof, wherein 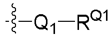 is selected from 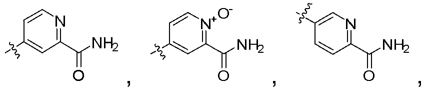 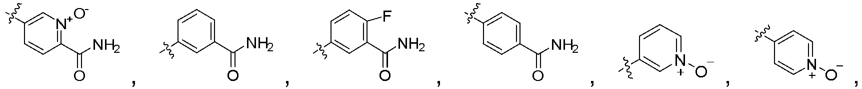 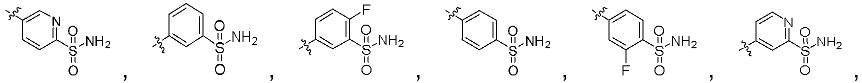 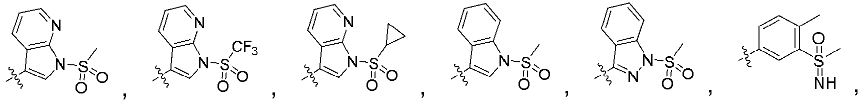 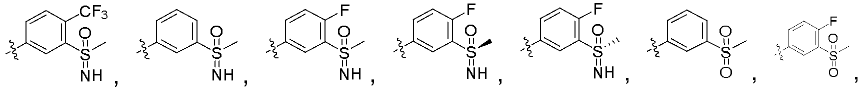 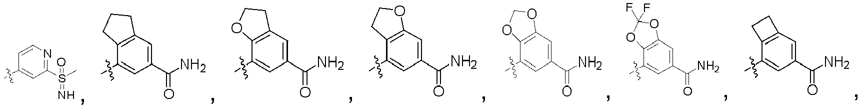 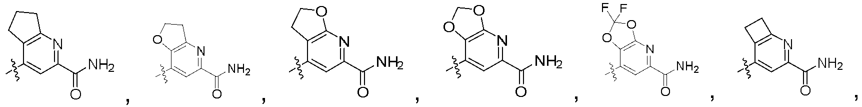 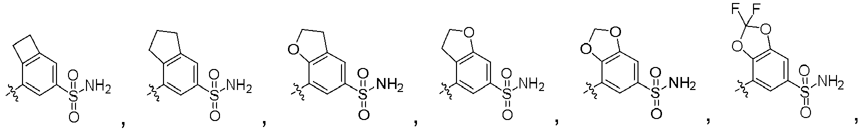 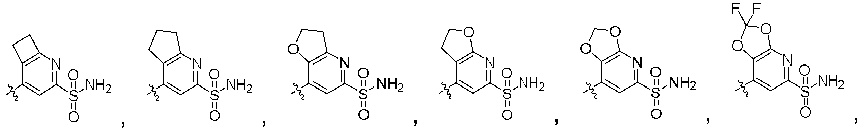 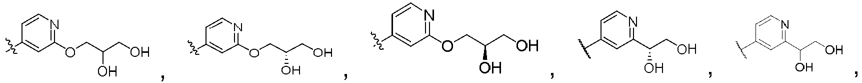 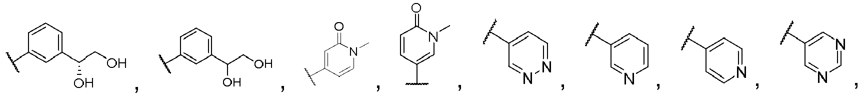 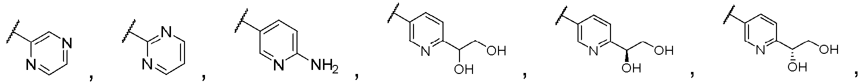 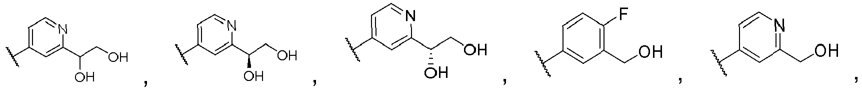 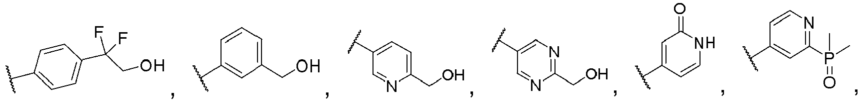 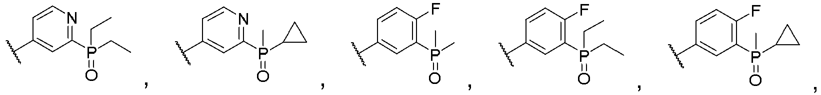 or 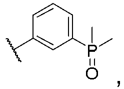 Q₁ being optionally substituted with 1 to 3 R^{q}'s;
B is selected from 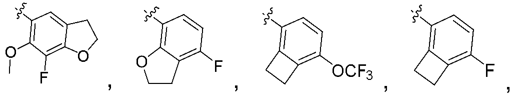 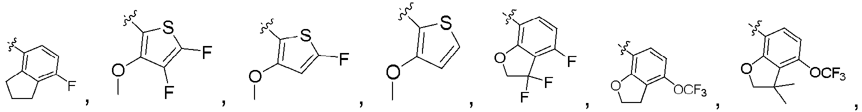 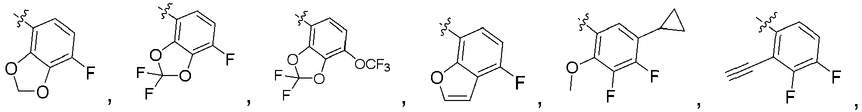 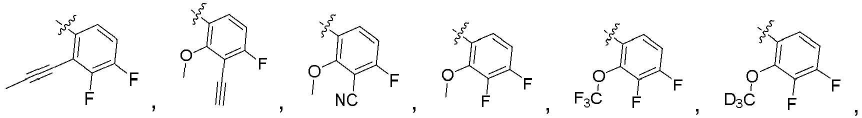 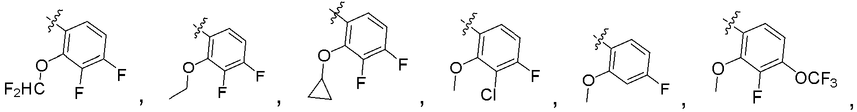 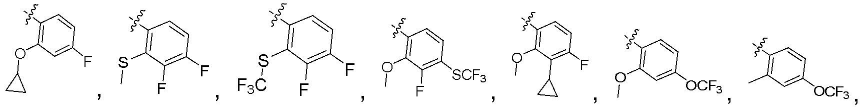 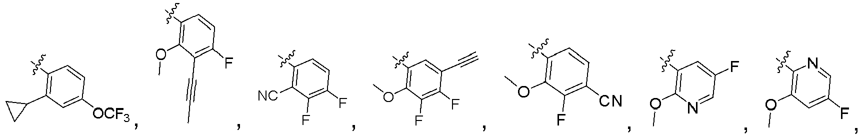 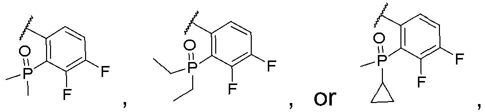 preferably 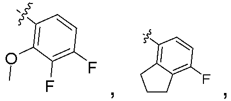 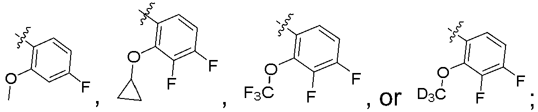 and
the other groups are as defined in the first, second, third, fourth, or fifth embodiment of the present invention.

The present invention relates to a compound as shown below or a stereoisomer, a deuterate, a solvate, a prodrug, a metabolite, a pharmaceutically acceptable salt, or a cocrystal thereof, wherein the compound is selected from one of the structures as shown in Table E below.

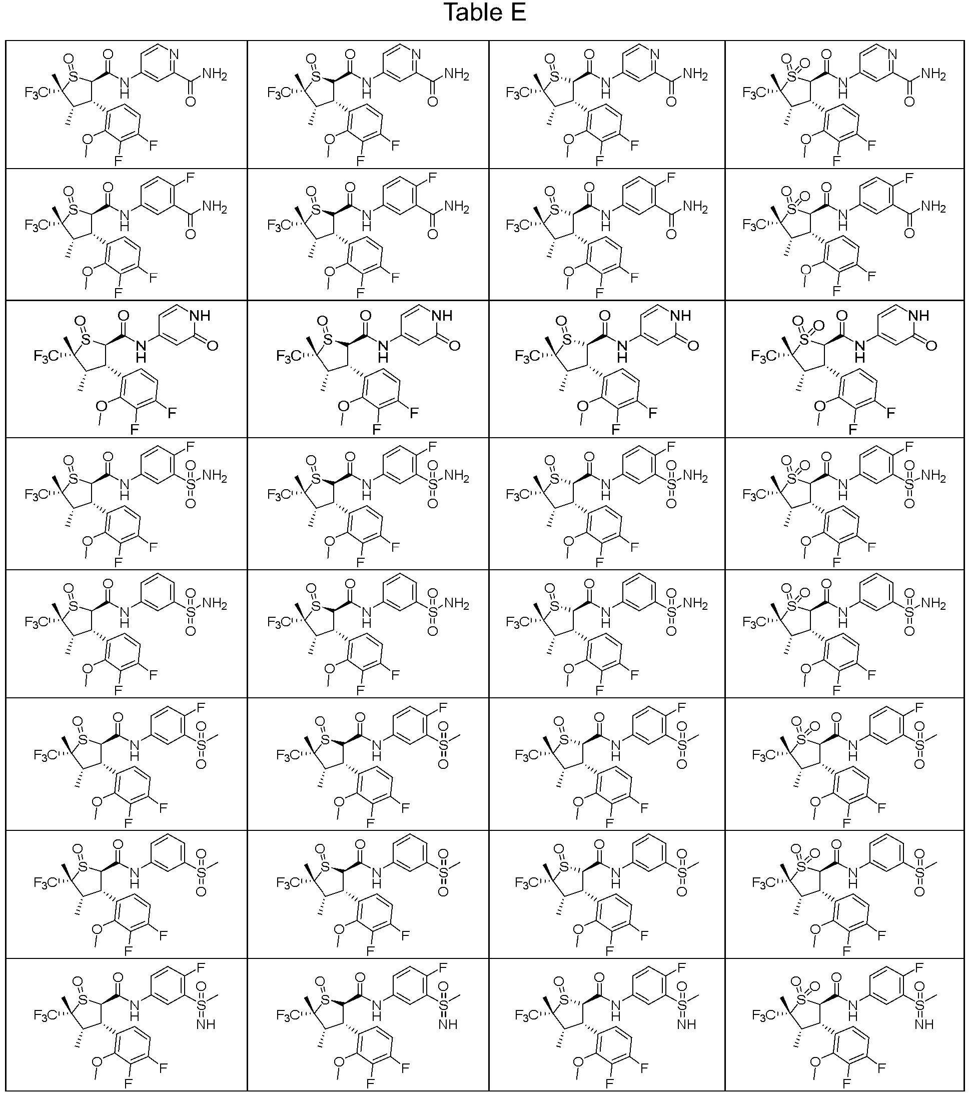

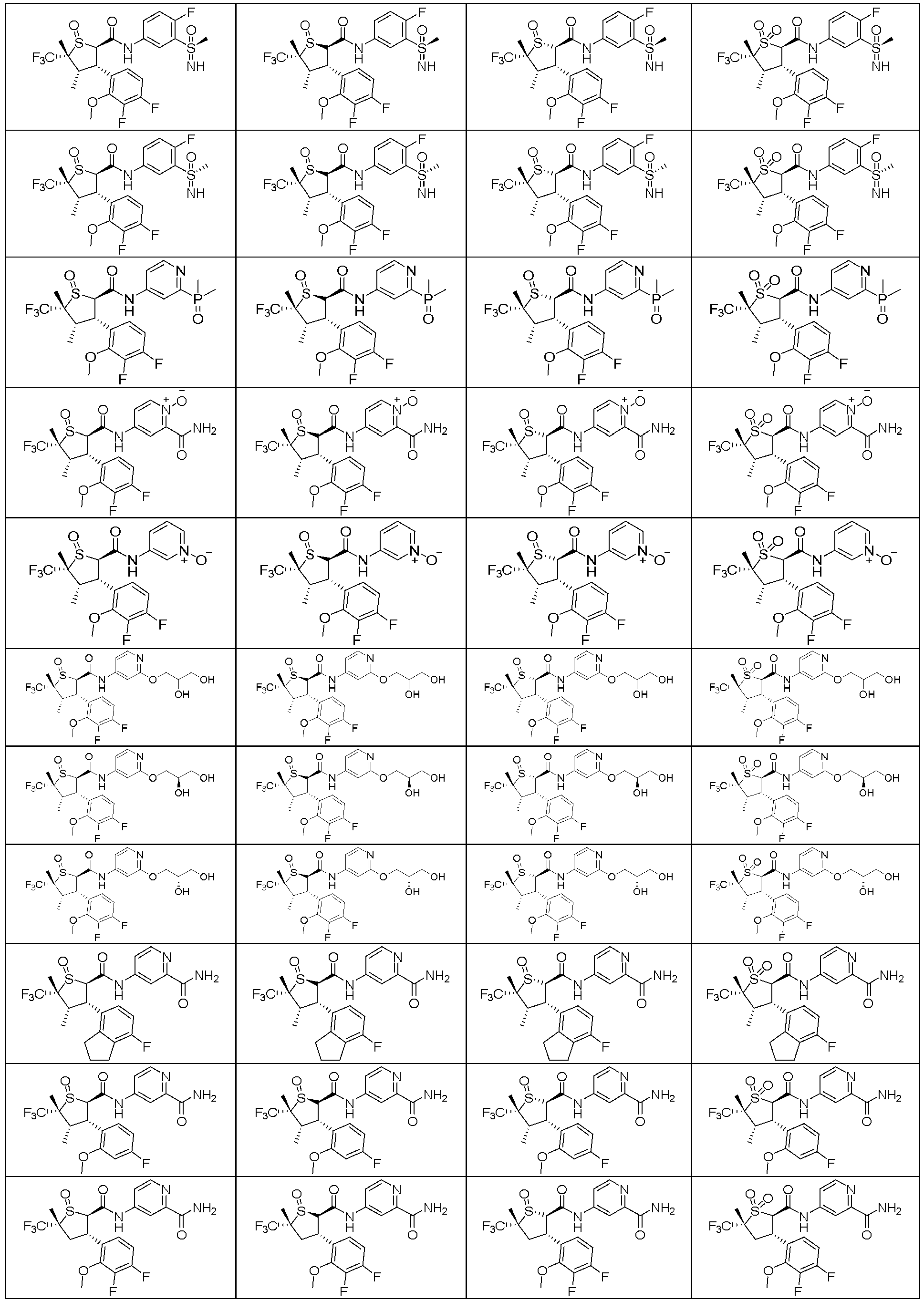

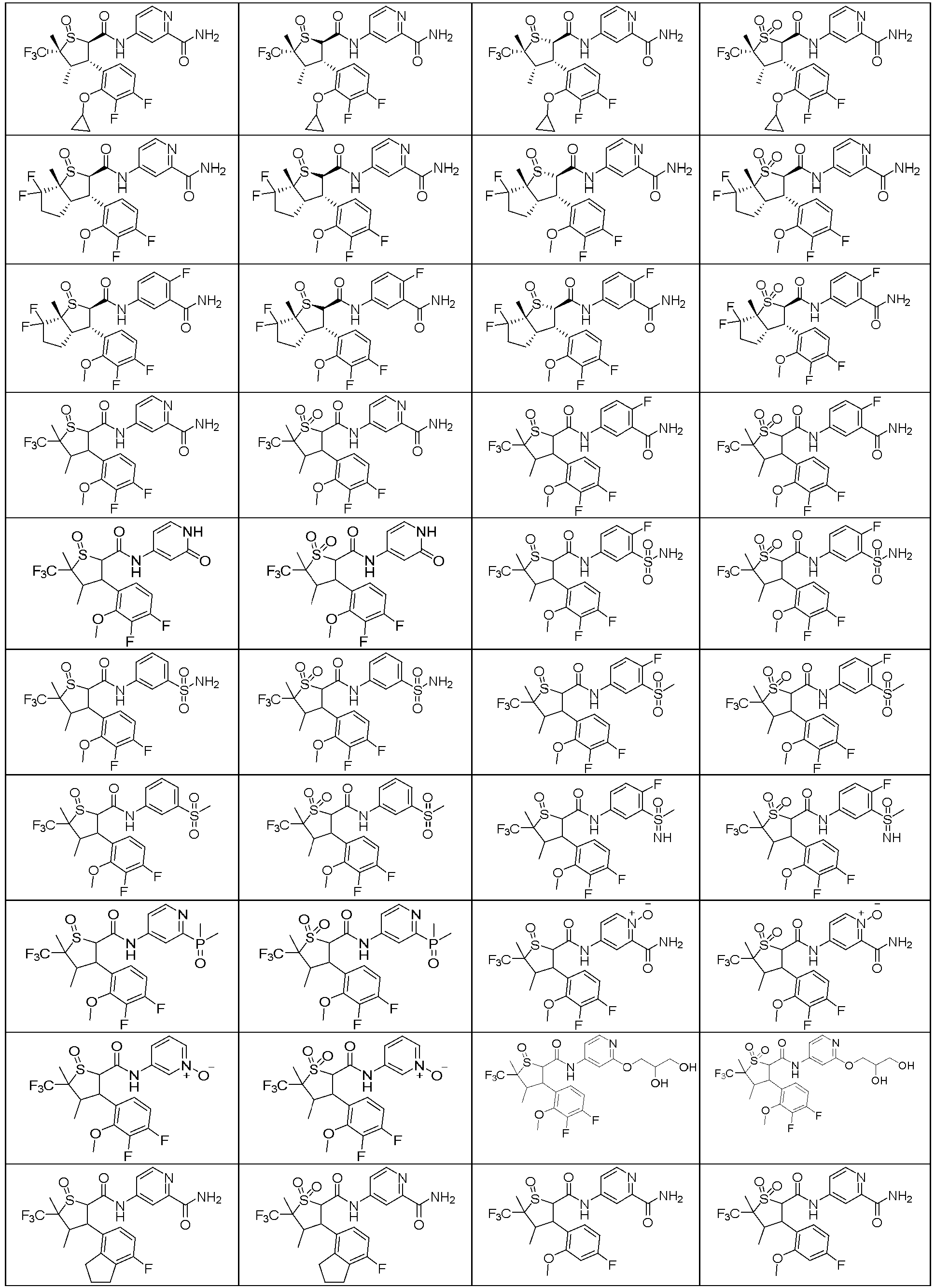

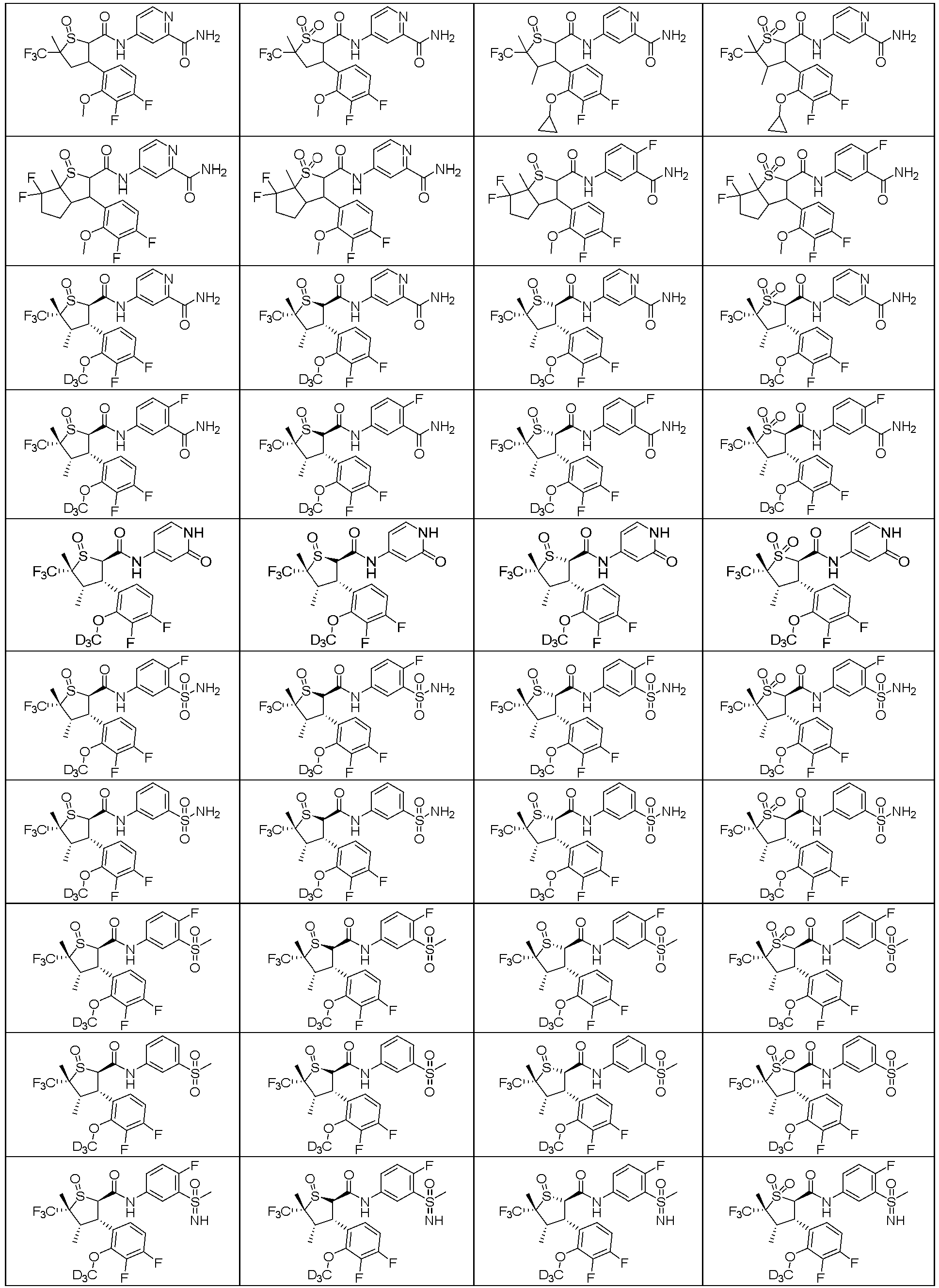

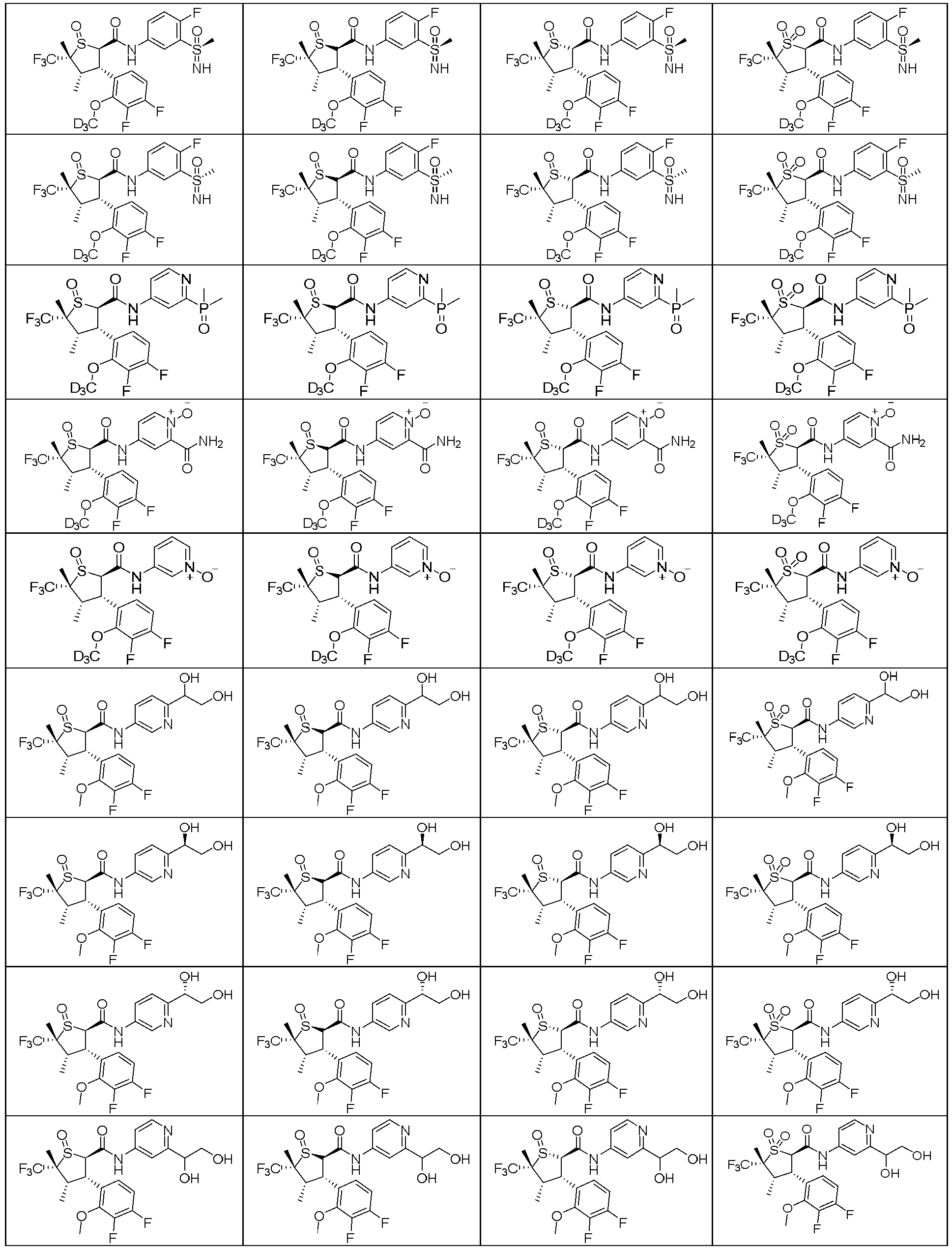

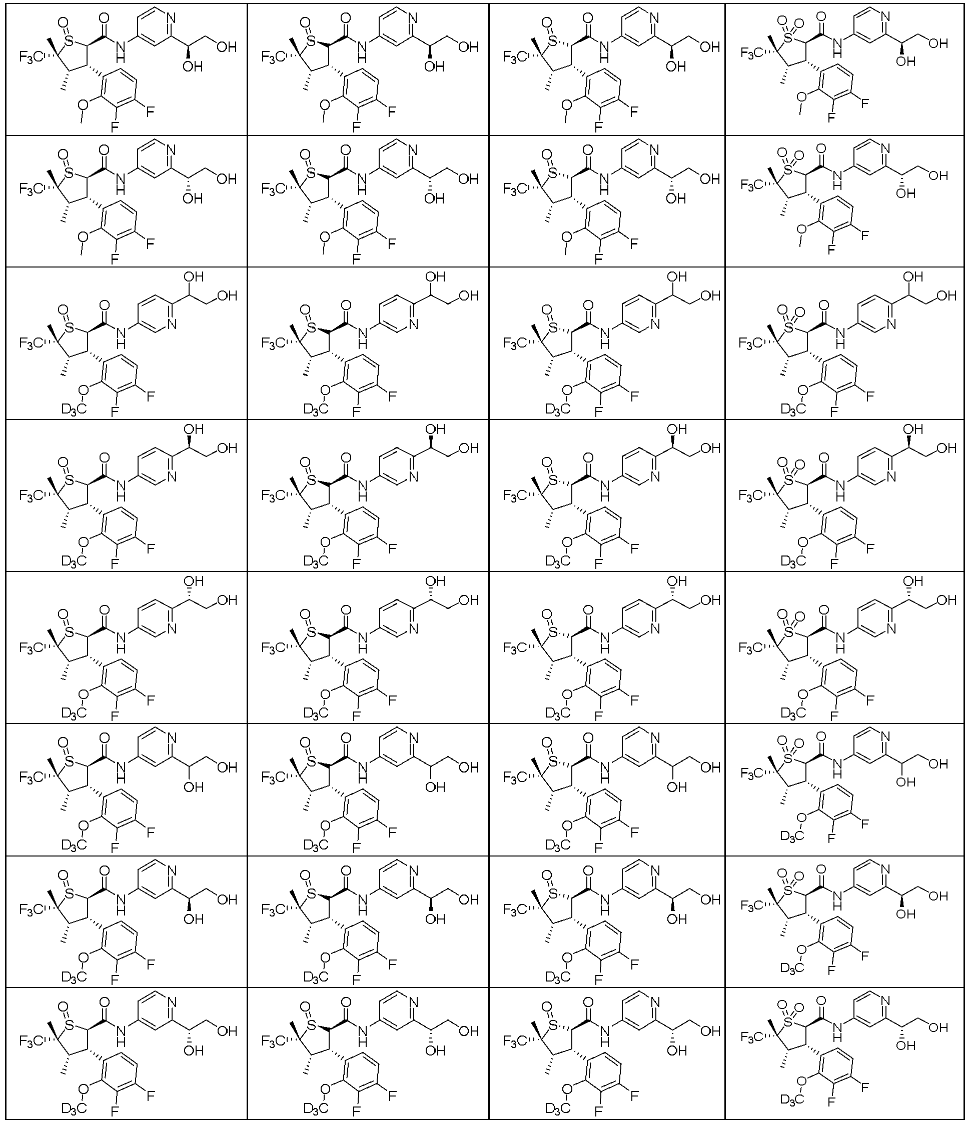

The present invention relates to a pharmaceutical composition comprising any of the above compound or a stereoisomer, a deuterate, a solvate, a prodrug, a metabolite, a pharmaceutically acceptable salt, or a cocrystal thereof, and a pharmaceutically acceptable carrier.

The present invention relates to a pharmaceutical composition comprising a therapeutically effective amount of the above compound or a stereoisomer, a deuterate, a solvate, a prodrug, a metabolite, a pharmaceutically acceptable salt, or a cocrystal thereof of the present invention, and a pharmaceutically acceptable carrier.

The present invention relates to a method for treating or alleviating a disease in a mammal, the method comprising administering to a subject a therapeutically effective amount of the above compound or a stereoisomer, a deuterate, a solvate, a prodrug, a metabolite, a pharmaceutically acceptable salt, or a cocrystal thereof, the therapeutically effective amount being preferably 1 mg to 1500 mg, and the disease being preferably pain.

In some embodiments, the pharmaceutical composition of the present invention may be in the form of a unit formulation (the amount of the main drug in the unit formulation is also referred to as "formulation specification").

As used herein, "effective amount" or "therapeutically effective amount" refers to the administration of a sufficient amount of the compound disclosed in the present application that will relieve to some extent one or more of the symptoms of the disease or condition being treated (e.g., treat or/and relieve pain). In some embodiments, the result is reduction and/or alleviation of a sign, symptom, or cause of the disease, or any other desired alteration to a biological system. For example, an "effective amount" for a therapeutic use is the amount comprising the compound disclosed in the present application required to provide a clinically significant decrease in a symptom of the disease. Examples of the therapeutically effective amount include, but not limited to, 1 mg to 1500 mg, 1 mg to 1200 mg, 1 mg to 1000 mg, 1 mg to 900 mg, 1 mg to 800 mg, 1 mg to 700 mg, 1 mg to 600 mg, 2 mg to 600 mg, 3 mg to 600 mg, 4 mg to 600 mg, 5 mg to 600 mg, 6 mg to 600 mg, 10 mg to 600 mg, 20 mg to 600 mg, 25 mg to 600 mg, 30 mg to 600 mg, 40 mg to 600 mg, 50 mg to 600 mg, 60 mg to 600 mg, 70 mg to 600 mg, 75 mg to 600 mg, 80 mg to 600 mg, 90 mg to 600 mg, 100 mg to 600 mg, 200 mg to 600 mg, 1 mg to 500 mg, 2 mg to 500 mg, 3 mg to 500 mg, 4 mg to 500 mg, 5 mg to 500 mg, 6 mg to 500 mg, 10 mg to 500 mg, 20 mg to 500 mg, 25 mg to 500 mg, 30 mg to 500 mg, 40 mg to 500 mg, 50 mg to 500 mg, 60 mg to 500 mg, 70 mg to 500 mg, 75 mg to 500 mg, 80 mg to 500 mg, 90 mg to 500 mg, 100 mg to 500 mg, 125 mg to 500 mg, 150 mg to 500 mg, 200 mg to 500 mg, 250 mg to 500 mg, 300 mg to 500 mg, 400 mg to 500 mg, 5 mg to 400 mg, 10 mg to 400 mg, 20 mg to 400 mg, 25 mg to 400 mg, 30 mg to 400 mg, 40 mg to 400 mg, 50 mg to 400 mg, 60 mg to 400 mg, 70 mg to 400 mg, 75 mg to 400 mg, 80 mg to 400 mg, 90 mg to 400 mg, 100 mg to 400 mg, 125 mg to 400 mg, 150 mg to 400 mg, 200 mg to 400 mg, 250 mg to 400 mg, 300 mg to 400 mg, 1 mg to 300 mg, 2 mg to 300 mg, 5 mg to 300 mg, 10 mg to 300 mg, 20 mg to 300 mg, 25 mg to 300 mg, 30 mg to 300 mg, 40 mg to 300 mg, 50 mg to 300 mg, 60 mg to 300 mg, 70 mg to 300 mg, 75 mg to 300 mg, 80 mg to 300 mg, 90 mg to 300 mg, 100 mg to 300 mg, 125 mg to 300 mg, 150 mg to 300 mg, 200 mg to 300 mg, 250 mg to 300 mg, 1 mg to 200 mg, 2 mg to 200 mg, 5 mg to 200 mg, 10 mg to 200 mg, 20 mg to 200 mg, 25 mg to 200 mg, 30 mg to 200 mg, 40 mg to 200 mg, 50 mg to 200 mg, 60 mg to 200 mg, 70 mg to 200 mg, 75 mg to 200 mg, 80 mg to 200 mg, 90 mg to 200 mg, 100 mg to 200 mg, 125 mg to 200 mg, 150 mg to 200 mg, 80 mg to 1000 mg, or 80 mg to 800 mg.

In some embodiments, the pharmaceutical composition comprises, but not limited to, 1 mg to 1000 mg, 20 mg to 800 mg, 40 mg to 800 mg, 40 mg to 400 mg, 25 mg to 200 mg, 1 mg, 5 mg, 10 mg, 15 mg, 20 mg, 25 mg, 30 mg, 35 mg, 40 mg, 45 mg, 50 mg, 55 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 300 mg, 320 mg, 400 mg, 480 mg, 500 mg, 600 mg, 640 mg, or 840 mg of the compound or a stereoisomer, a deuterate, a solvate, a prodrug, a metabolite, a pharmaceutically acceptable salt, or a cocrystal thereof of the present invention.

A method for treating a disease in a mammal, the method comprising administering to a subject a therapeutically effective amount of the compound or a stereoisomer, a deuterate, a solvate, a prodrug, a metabolite, a pharmaceutically acceptable salt, or a cocrystal thereof of the present invention, the therapeutically effective amount being preferably 1 mg to 1500 mg, and the disease being preferably treating or alleviating pain.

A method for treating or alleviating a disease in a mammal, the method comprising administering a drug, i.e., the compound or a stereoisomer, a deuterate, a solvate, a prodrug, a metabolite, a pharmaceutically acceptable salt, or a cocrystal thereof of the present invention to a subject at a daily dose of 1 mg to 1000 mg per day. The daily dose may be a single dose or divided doses. In some embodiments, the daily dose includes, but not limited to, 10 mg to 1500 mg per day, 10 mg to 1000 mg per day, 10 mg to 800 mg per day, 25 mg to 800 mg per day, 50 mg to 800 mg per day, 100 mg to 800 mg per day, 200 mg to 800 mg per day, 25 mg to 400 mg per day, 50 mg to 400 mg per day, 100 mg to 400 mg per day, or 200 mg to 400 mg per day, and in some embodiments, the daily dose includes, but not limited to, 10 mg per day, 20 mg per day, 25 mg per day, 50 mg per day, 80 mg per day, 100 mg per day, 125 mg per day, 150 mg per day, 160 mg per day, 200 mg per day, 300 mg per day, 320 mg per day, 400 mg per day, 480 mg per day, 600 mg per day, 640 mg per day, 800 mg per day, or 1000 mg per day.

The present invention relates to a kit. The kit may include a composition in the form of a single dose or multiple doses. The kit comprises the compound or a stereoisomer, a deuterate, a solvate, a prodrug, a metabolite, a pharmaceutically acceptable salt, or a cocrystal thereof of the present invention, the amount of the compound or a stereoisomer, a deuterate, a solvate, a prodrug, a metabolite, a pharmaceutically acceptable salt, or a cocrystal thereof of the present invention being the same as the amount thereof in the above pharmaceutical composition.

The present invention relates to use of any of the above compound or a stereoisomer, a deuterate, a solvate, a prodrug, a metabolite, a pharmaceutically acceptable salt, or a cocrystal thereof in the manufacture of a medicament for treating or/and alleviating pain.

The present invention relates to use of the above pharmaceutical composition in the manufacture of a medicament for treating or/and alleviating pain.

The amount of the compound or a stereoisomer, a deuterate, a solvate, a prodrug, a metabolite, a pharmaceutically acceptable salt, or a cocrystal thereof of the present invention is calculated as a free base in every instance.

Unless otherwise stated, the terms used in the description and claims have the following meanings.

The carbon, hydrogen, oxygen, sulfur, nitrogen, F, Cl, Br, and I involved in the groups and compounds of the present invention all include their isotopes, and the carbon, hydrogen, oxygen, sulfur, or nitrogen involved in the groups and compounds of the present invention is optionally replaced by one or more of their corresponding isotopes. The isotopes of carbon include¹²C, ¹³C, and ¹⁴C, the isotopes of hydrogen include protium (H), deuterium (D, also known as heavy hydrogen), and tritium (T, also known as superheavy hydrogen), the isotopes of oxygen include¹⁶O, ¹⁷O, and ¹⁸O. The isotopes of sulfur include ³²S, ³³S, ³⁴S, and ³⁶S, the isotopes of nitrogen include ¹⁴N and ¹⁵N, the isotopes of fluorine include ¹⁷F and ¹⁹F, the isotopes of chlorine include ³⁵Cl and ³⁷Cl, and the isotopes of bromine include ⁷⁹Br and ⁸¹Br.

"Halogen" refers to F, Cl, Br, or I.

"Halogen-substituted" refers to being substituted by F, Cl, Br, or I, including but not limited to, being substituted by 1 to 10 substituents selected from F, Cl, Br, or I, being substituted by 1 to 6 substituents selected from F, Cl, Br, or I, or being substituted by 1 to 4 substituents selected from F, CI, Br, or I. "Halogen-substituted" is abbreviated as "halo".

"Alkyl" refers to a substituted or unsubstituted, linear or branched, saturated aliphatic hydrocarbon group, including, but not limited to, alkyl group of 1 to 20 carbon atoms, alkyl group of 1 to 8 carbon atoms, alkyl group of 1 to 6 carbon atoms, and alkyl group of 1 to 4 carbon atoms. Non-limiting examples include methyl, ethyl, n-propyl, isopropyl, n-butyl, sec-butyl, neo-butyl, tert-butyl, n-pentyl, isopentyl, neo-pentyl, n-hexyl, and various branched isomers thereof. The definition of the alkyl group appearing herein is consistent with this definition. The alkyl group may be monovalent, divalent, trivalent, or tetravalent.

"Alkylene" refers to a substituted or unsubstituted, linear or branched, divalent saturated hydrocarbon group, including -(CH₂)ᵥ- (v is an integer of 1 to 10). Examples of the alkylene group include, but not limited to, methylene, ethylene, propylene, butylene, etc.

"Cycloalkyl" refers to a substituted or unsubstituted, saturated carbocyclic hydrocarbon group, typically having 3 to 10 carbon atoms. Non-limiting examples include cyclopropyl, cyclobutyl, cyclopentyl, cyclohexyl, or cycloheptyl, etc. The definition of the cycloalkyl group appearing herein is as described above. The cycloalkyl group may be monovalent, divalent, trivalent, or tetravalent.

"Alkenyl" refers to a substituted or unsubstituted, linear or branched, unsaturated hydrocarbon group having at least 1, usually 1, 2, or 3 carbon-carbon double bonds, and the backbone includes, but not limited to, 2 to 10, 2 to 6, or 2 to 4 carbon atoms. Examples of the alkenyl group include, but not limited to, ethenyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, and 1,4-hexadiene. The definition of the alkenyl group appearing herein is consistent with this definition. The alkenyl group may be monovalent, divalent, trivalent, or tetravalent.

"Alkynyl" refers to a substituted or unsubstituted, straight or branched, unsaturated hydrocarbon group having at least 1, typically 1, 2, or 3, carbon-carbon triple bonds, including, but not limited to, comprising 2 to 10 carbon atoms, 2 to 6 carbon atoms, or 2 to 4 carbon atoms in the backbone. Examples of the alkynyl group include, but not limited to, ethynyl, propargyl, 1-propynyl, 2-propynyl, 1-butynyl, 2-butynyl, 3-butynyl, 1-pentynyl, 2-pentynyl, 3-pentynyl, 4-pentynyl, 1-methyl-1-butynyl, 2-methyl-1-butynyl, 2-methyl-3-butynyl, 1-hexynyl, 2-hexynyl, 3-hexynyl, 4-hexynyl, 5-hexynyl, 1-methyl-1-pentynyl, 2-methyl-1-pentynyl, 1-heptynyl, 2-heptynyl, 3-heptynyl, 4-heptynyl, 1-octynyl, 3-octynyl, 1-nonynyl, 3-nonynyl, 1-decynyl, 4-decynyl, etc. The alkynyl group may be monovalent, divalent, trivalent, or tetravalent.

"Alkoxy" refers to a substituted or unsubstituted -O-alkyl group. Non-limiting examples include methoxy, ethoxy, n-propoxy, isopropoxy, n-butoxy, sec-butoxy, tert-butoxy, n-pentoxy, n-hexoxy, cyclopropoxy, and cyclobutoxy.

"Carbocyclyl" or "carbocycle" refers to a substituted or unsubstituted, saturated or unsaturated, aromatic or non-aromatic ring, wherein the aromatic or non-aromatic ring may be a 3- to 8-membered monocyclic ring system, a 4-to 12-membered bicyclic ring system, or a 10- to 15-membered tricyclic ring system, the carbocyclyl group may be attached to the aromatic or non-aromatic ring, and the aromatic or non-aromatic ring is optionally a monocyclic ring, a bridged ring, or a spiro ring. Non-limiting examples include cyclopropane, cyclobutane, cyclopentane, cyclohexane, cycloheptane, 1-cyclopentyl-1-alkenyl, 1-cyclopentyl-2-alkenyl, 1-cyclopentyl-3-alkenyl, cyclohexyl, 1-cyclohexyl-2-alkenyl, 1-cyclohexyl-3-alkenyl, cyclohexenyl, benzene ring, naphthalene ring, 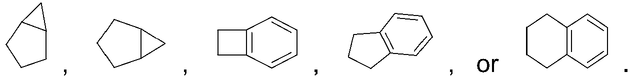 "Carbocyclyl" or "carbocycle" may be monovalent, divalent, trivalent, or tetravalent.

"Heterocyclyl" or "heterocycle" refers to a substituted or unsubstituted, saturated or unsaturated, aromatic or non-aromatic ring, wherein the aromatic or non-aromatic ring may be a 3- to 8-membered monocyclic ring system, a 4- to 12-membered bicyclic ring system, or a 10- to 15-membered tricyclic ring system, and contains one or more (including but not limited to 2, 3, 4, or 5) heteroatoms selected from N, O, or S, and the N and S selectively substituted in the ring of the heterocyclyl group may be oxidized to various oxidation states. The heterocyclyl group may be attached to a heteroatom or a carbon atom, the heterocyclyl group may be attached to an aromatic ring or a non-aromatic ring, and the heterocyclyl group may be attached with a bridged ring or a spiro ring. Non-limiting examples include oxiranyl, aziridinyl, oxetanyl, azetidinyl, 1,3-dioxolanyl, 1,4-dioxolanyl, 1,3-dioxanyl, azepanyl, pyridyl, furyl, thienyl, pyranyl, N-alkylpyrrolyl, pyrimidinyl, pyrazinyl, pyridazinyl, imidazolyl, piperidinyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, tetrahydrothiazolyl, tetrahydropyranyl, benzimidazolyl, benzopyridyl, pyrrolopyridyl, benzodihydrofuryl, pyrrolyl, pyrazolyl, thiazolyl, oxazolyl, pyrazinyl, indazolyl, benzothienyl, benzofuranyl, benzopyrrolyl, benzimidazolyl, benzothiazolyl, benzoxazolyl, benzopyridyl, benzopyrimidinyl, benzopyrazinyl, piperazinyl, azabicyclo[3.2.1]octanyl, azabicyclo[5.2.0]nonanyl, oxatricyclo[5.3.1.1]dodecanyl, azaadamantyl, oxaspiro[3.3]heptanyl, 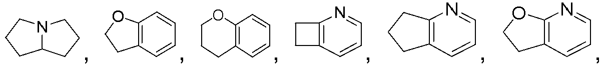 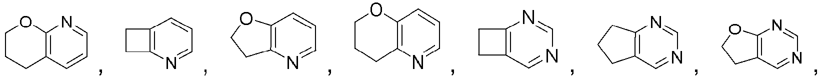 or 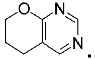 "Heterocyclyl" or "heterocycle" may be monovalent, divalent, trivalent, or tetravalent.

"Heteroaryl" or "heteroaromatic ring" refers to a substituted or unsubstituted aromatic hydrocarbon group containing 1 to 5 optional heteroatoms or heteroatom-containing groups (including but not limited to N, O, or S(=O)n, where n is 0, 1, or 2). The number of ring atoms in the heteroaromatic ring includes but not limited to 5 to 15, 5 to 10, or 5 to 6. Non-limiting examples of the heteroaryl group include, but not limited to, pyridyl, furyl, thienyl, pyridinyl, pyranyl, N-alkylpyrrolyl, pyrimidinyl, pyrazinyl, pyridazinyl, imidazolyl, benzopyrazole, benzimidazole, benzopyridine, pyrrolopyridine, etc. The heteroaryl ring may be fused to a saturated or unsaturated carbocyclic or heterocyclic ring, wherein the ring attached to a parent structure is the heteroaryl ring, and non-limiting examples include 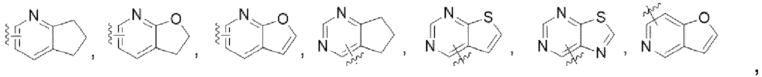 and 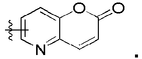 The definition of the heteroaryl group appearing herein is consistent with this definition. The heteroaryl group may be monovalent, divalent, trivalent, or tetravalent. When divalent, trivalent, or tetravalent, the point of attachment is on the heteroaryl ring.

"Substitution" or "substituted" refers to being substituted by one or more (including but not limited to 2, 3, 4, or 5) substituents, including but not limited to H, F, Cl, Br, I, alkyl, cycloalkyl, alkoxy, haloalkyl, thiol, hydroxyl, nitro, mercapto, amino, cyano, isocyano, aryl, heteroaryl, heterocyclyl, bridged cyclic group, spiro cyclic group, fused cyclic group, hydroxyalkyl, =O, carbonyl, aldehyde, carboxylic acid, formate ester, -(CH)₂)ₘ-C(=O)-R^{a}, -O-(CH₂)ₘ-C(=O)-R^{a}, -(CH₂)ₘ-C(=O)-NR^{b}R^{c}, -(CH₂)ₘS(=O)ₙR^{a}, -(CH₂)ₘ-alkenyl-R^{a}, OR^{d}, or -(CH)₂)ₘ-alkynyl-R^{a}(where m and n are 0, 1, or 2), arylthio, thiocarbonyl, silyl, or -NR^{b}R^{c}, etc., wherein R^{b} and R^{c} are independently selected from H, hydroxy, amino, carbonyl, alkyl, alkoxy, cycloalkyl, heterocyclyl, aryl, heteroaryl, sulfonyl, or trifluoromethanesulfonyl, or alternatively, R^{b} and R^{c} may form a five- or six-membered cycloalkyl or heterocyclic group, and R^{a} and R^{d} are each independently selected from aryl, heteroaryl, alkyl, alkoxy, cycloalkyl, heterocyclyl, carbonyl, ester group, bridged cyclic group, spiro cyclic group, or fused cyclic group.

"Substituted with one to X substituents selected from..." means being substituted with 1, 2, 3, ..., X substituents selected from..., X being any integer selected from 1 to 10. For example, "substituted with 1 to 4 R^{k}'s" means being substituted with 1, 2, 3 or 4 R^{k}'s. For example, "substituted with 1 to 5 substituents selected from..." means being substituted with 1, 2, 3, 4, or 5 substituents selected from... For example, "a hetero-bridged cyclic ring optionally substituted with 1 to 4 substituents selected from D or F" means that the hetero-bridged cyclic ring is optionally substituted with 1, 2, 3, or 4 substituents selected from D or F.

"X- to Y-membered ring" (where X is an integer, and 3 ≤ X < Y, and Y is selected from any integer between 4 and 12) includes X-, X+1-, X+2-, X+3-, X+4-..., and Y-membered rings. The ring includes heterocyclic ring, carbocyclic ring, aromatic ring, aryl, heteroaryl, cycloalkyl, heteromonocyclic ring, heterofused ring, heterospirocyclic ring, or hetero-bridged cyclic ring. For example, "4- to 7-membered heteromonocyclic ring" refers to a 4-, 5-, 6-, or 7-membered heteromonocyclic ring, and "5- to 10-membered heterofused ring" refers to a 5-, 6-, 7-, 8-, 9-, or 10-membered heterofused ring.

Unless otherwise indicated, a wedged solid bond ( 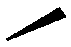 ) and a wedged dashed bond ( 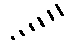 ) are used to indicate the absolute configuration of a stereocenter, and a straight solid bond ( 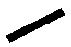 ) and a straight dashed bond ( 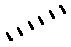 ) are used to indicate the relative configuration of stereocenters.

"Optional" or "optionally" means that the subsequently described event or circumstance may but need not occur, and that the description includes instances where the event or circumstance occurs and instances where it does not. For example: "alkyl optionally substituted with F" means that the alkyl may, but not necessarily, be substituted with F, and the description includes the case where the alkyl is substituted with F and the case where the alkyl is not substituted with F.

"Pharmaceutically acceptable salt" or "pharmaceutically acceptable salt thereof" refers to a salt that retains the biological effectiveness and properties of the free acid or free base of the compound of the present invention, the salt being obtained by reacting the free acid with a non-toxic inorganic or organic base, or reacting the free base with a non-toxic inorganic or organic acid.

"Pharmaceutical composition" refers to a mixture formed by one or more of the compound or a stereoisomer, a tautomer, a deuterate, a solvate, a prodrug, a metabolite, a pharmaceutically acceptable salt, or a cocrystal thereof of the present invention with another chemical component, wherein the "another chemical component" refers to a pharmaceutically acceptable carrier, an excipient, and/or one or more other therapeutic agents.

"Carrier" refers to a material that does not cause significant irritation to an organism and does not abrogate the biological activity and properties of the administered compound.

"Formulation specification" refers to the weight of the main drug contained in one capsule, tablet or other unit formulation. "Prodrug" means being able to be converted to the biologically active compound of the present invention by in vivo metabolism. The prodrug of the present invention is prepared by modifying an amino or carboxyl group in the compound of the present invention, and the modification can be removed by an conventional operation, or in vivo, to obtain the parent compound. When the prodrug of the present invention is administered to a mammalian individual, the prodrug is cleaved to form a free amino or carboxyl group.

"Cocrystal" refers to a crystal formed by the combination of an active pharmaceutical ingredient (API) and a cocrystal former (CCF) under the action of hydrogen bonds or other non-covalent bonds, wherein the API and the CCF in a pure state are both solid at room temperature, and there is a fixed stoichiometric ratio between the components. The cocrystal is a multicomponent crystal, including both a binary cocrystal formed between two neutral solids and a multiple cocrystal formed between a neutral solid and a salt or solvate.

"Animal" is meant to include mammals, such as humans, companion animals, zoo animals, and farm animals, preferably humans, horses, or dogs.

"Stereoisomer" refers to isomers resulting from different spatial arrangements of atoms in a molecule, including cis-trans isomers, enantiomers, and conformers.

"Tautomer" refer to functional group isomers produced by the rapid movement of an atom between two positions in a molecule, such as keto-enol tautomerism, amide-imidol tautomerism, etc.

### DETAILED DESCRIPTION OF THE EMBODIMENTS

The following examples illustrate the technical solutions of the present invention in detail, but the scope of protection of the present invention includes but is not limited thereto.

The structure of compounds was determined by nuclear magnetic resonance (NMR) or/and mass spectrometry (MS). NMR shift (δ) was given in unit of 10⁻⁶ (ppm). NMR determination was performed using Bruker Avance III 400 and Bruker Avance 300 nuclear magnetic apparatus, the solvents for determination were deuterated dimethyl sulfoxide (DMSO-d₆), deuterated chloroform (CDCl₃), and deuterated methanol (CD₃OD), and the internal standard was tetramethylsilane (TMS).

MS determination was performed using Agilent 6120B (ESI) and Agilent 6120B (APCI).

HPLC determination was performed using an Agilent 1260DAD high pressure liquid chromatograph (Zorbax SB-C18 100×4.6 mm, 3.5 µM);
Yantai Huanghai HSGF254 or Qingdao GF254 silica gel plates were used as thin-layer chromatography silica gel plates. The specification used for the silica gel plates used in thin-layer chromatography (TLC) was 0.15 mm to 0.20 mm, and the specification used for isolation and purification of products by thin-layer chromatography was 0.4 mm to 0.5 mm.

Yantai Huanghai 200- to 300-mesh silica gel was generally used as a carrier for column chromatography.

For the purposes of the present invention, compounds used in the reactions described herein were prepared starting from commercially available chemicals and/or compounds described in chemical literature and using organic synthesis techniques known to a person skilled in the art. "Commercially available chemicals" were obtained from standard commercial sources, including Shanghai Aladdin Biochemical Technology Co., Ltd., Shanghai Macklin Biochemical Technology Co., Ltd., Sigma-Aldrich, Alfa Aesar (China) Chemical Co., Ltd., TCI (Shanghai) Chemical Industrial Development Co., Ltd., Energy Chemical, Shanghai Titan Technology Co., Ltd., Kelon Chemical, J&K Scientific Co., Ltd., etc.

T3P: 1-n-propyl phosphoric anhydride; DMF: N,N-dimethylformamide; TEA: triethanolamine; DIPEA: N,N-diisopropylethylamine.
abs1/abs2 represents that the chiral center is in an unknown single configuration.

### Example 1: Preparation of Compound 1-3a and Compound 1-3b

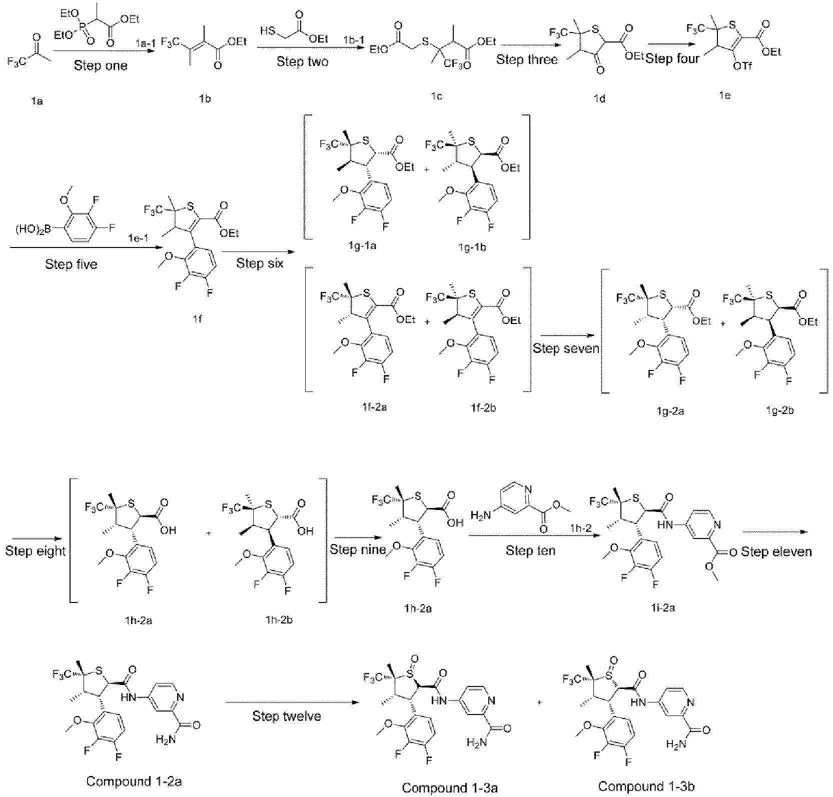

### Step one: Preparation of Compound 1b

In an ice bath, 1a-1 (30.96 g, 129.95 mmol) was dissolved in tetrahydrofuran (130 mL), and sodium hydride (5.20 g, 130 mmol) was added in batches. The mixture was reacted under a nitrogen atmosphere in the ice bath for 30 minutes. Then 1a (11.20 g, 100 mmol) was dissolved in tetrahydrofuran (20 mL), and the solution was added dropwise to the reaction system. The mixture was allowed to naturally warm to room temperature and reacted for 18 h under a nitrogen atmosphere. In the ice bath, 1 M hydrochloric acid was slowly added dropwise to the reaction system until pH = 7 to 8, and the reaction was extracted with ethyl ether (150 mL×3). The organic phases were combined, dried over anhydrous sodium sulfate, filtered, and concentrated (concentrated at normal temperature) to obtain a crude product. The crude product was purified by silica gel column chromatography, affording 1b (14.1 g, yield 71.88%, a mixture of E/Z configurations).

### Step two: Preparation of Compound 1c

In the ice bath, 1b-1 (8.63 g, 71.88 mmol) was added to a round bottom flask, piperidine (1.22 g, 14.38 mmol) was added dropwise to the system, 1b (6.0 g, 71.88 mmol) was added dropwise, and the mixture was reacted at 50°C for 24 h under nitrogen protection. In the ice bath, 0.1 M hydrochloric acid (100 mL) was added to quench the reaction, and the reaction was extracted with ethyl ether (100 mL×3). The organic phases were combined, dried over anhydrous sodium sulfate, filtered, and concentrated (concentrated at 25°C) to obtain a crude product of 1c. The crude product was purified by silica gel column chromatography, affording 1c (6.3 g, yield 27.96%).

LC-MS m/z = 317.1 [M+H]⁺

### Step three: Preparation of Compound 1d

In the ice bath, 1c (6.3 g, 19.92 mmol) was dissolved in ethyl ether (120 mL). Under nitrogen protection, potassium tert-butoxide (2.91 g, 25.90 mmol) was slowly added dropwise to the system, and the system was reacted in the ice bath for 2 h. In the ice bath, glacial acetic acid (1.56 mL) and water (100 mL) were added to the reaction system to quench the reaction, and the reaction was extracted with ethyl ether (100 mL×3). The organic phases were combined, dried over anhydrous sodium sulfate, filtered, and concentrated, affording 1d.

### Step four: Preparation of Compound 1e

1d (4.05 g, 15 mmol) was dissolved in dichloromethane (40 mL), and the solution was pre-cooled at -78°C for 15 minutes. Under a nitrogen atmosphere, N,N-diisopropylethylamine (2.32 g, 17.99 mmol) was added dropwise, and trifluoromethanesulfonic anhydride (4.23 g, 15 mmol) in dichloromethane (10 mL) was slowly added dropwise to the system. After the addition was completed, a reaction was allowed to proceed further at -78°C for 2 h. In the ice bath, a saturated sodium bicarbonate aqueous solution (50 mL) was slowly added to the reaction system to quench the reaction, and the reaction was extracted with dichloromethane (50 mL×3). The organic phases were combined, dried over anhydrous sodium sulfate, filtered, and concentrated to obtain a crude product. The crude product was purified by silica gel column chromatography, affording 1e (5.2 g, yield 86.16%).

### Step five: Preparation of Compound 1f

1e (5.2 g, 12.92 mmol) was dissolved in toluene (50 mL), and 1e-1 (2.67 g, 14.21 mmol) and Pd(PPh₃)₄ (0.75 g, 0.65 mmol) were added successively. Potassium phosphate (8.23 g, 38.71 mmol) was formulated into a 2 M aqueous solution and added to the system. The mixture was reacted at 100°C for 6 h under a nitrogen atmosphere. The reaction was cooled to room temperature. The reaction system was filtered, and the filter cake was washed with ethyl acetate (10 mL×2). The filtrate was allowed to separate into layers, the organic phase was collected, and the aqueous phase was extracted with ethyl acetate (30 mL×3). The organic phases were combined, dried over anhydrous sodium sulfate, filtered, and concentrated to obtain a crude product. The crude product was purified by silica gel column chromatography, affording 1f (5.1 g, yield 99.59%).

LC-MS m/z = 397.1 [M+H]⁺

### Step six: Preparation of Compounds 1f-2a and 1f-2b

1f (3.7 g, 9.33 mmol) was dissolved in methanol (50 mL), and Pd/C (1.99 g, 18.56 mmol) was added. After the addition was completed, the pressure was increased to 2 MPa under a hydrogen atmosphere, and a reaction was allowed to proceed at room temperature for 24 h. The reaction was filtered, and the filtrate was concentrated to obtain a crude product. The crude product was purified by silica gel column chromatography, affording a mixture of 1g-1a and 1g-1b (1.02 g, yield 27.44%). Also, a mixture of unreacted configuration starting materials 1f-2a and 1f-2b (0.148 g, yield 4.00%) were recovered.

### Step seven: Preparation of Compounds 1g-2a and 1g-2b

The mixture of 1f-2a and 1f-2b (0.148 g, 0.38 mmol) was dissolved in methanol (10 mL), and palladium/carbon (0.15 g, 1.41 mmol) was added. After the addition was completed, the pressure was increased to 2.5 MPa under a hydrogen atmosphere, and a reaction was allowed to proceed at 90°C for 24 h. The reaction was filtered, and the filtrate was concentrated to obtain a crude product. The crude product was purified by silica gel column chromatography, affording a mixture of 1g-2a and 1g-2b (0.052 g, yield 34.35%).

### Step eight: Preparation of Compounds 1h-2a and 1h-2b

In the ice bath and under a nitrogen atmosphere, the mixture of 1g-2a and 1g-2b (0.052 g, 0.13 mmol) was dissolved in tetrahydrofuran (5 mL). The system was pre-cooled for 15 minutes, and potassium tert-butoxide (0.048 g, 0.43 mmol) was slowly added dropwise to the system (internal temperature < 13°C). After the addition was completed, a reaction was allowed to proceed in the ice bath for 2 h. In the ice bath, 1 N hydrochloric acid (internal temperature < 13°C) was slowly added dropwise to the system until pH = 1. Water (5 mL) was added, and the mixture was extracted with ethyl acetate (10 mL×3). The organic phases were combined, dried over anhydrous sodium sulfate, filtered, and concentrated, affording a mixture of 1h-2a and 1h-2b.

LC-MS m/z = 369.0 [M-H]⁻

### Step nine: Preparation of Compound 1h-2a

3.0 g of the mixture of Compounds 1h-2a and 1h-2b was subjected to SFC preparation and freeze dried, affording Compound 1h-2a (1.28 g, chiral HPLC retention time: 0.760 min) and Compound 1h-2b (1.11 g, chiral HPLC retention time: 0.966 min). Chiral HPLC determination method: (instrument: SHIMADZU LC-30 AD, chiral column: Chiralcel IG column. Sample-making method: the crude product was dissolved in acetonitrile to make a sample solution. Mobile phase system: carbon dioxide/0.05% DEA in ethanol. Elution gradient: 5% to 40%; elution time: 3 min).

Conditions for SFC preparation: instrument: Waters 150 Prep-SFC A was used, preparation column: Chiralcel IG column. Sample-making method: the crude product was dissolved in acetonitrile to make a sample solution having a concentration of 2 mg/mL. Mobile phase system: carbon dioxide/ethanol, ethanol content 10%; flow rate: 100 mL/min, elution time: 2 min.

### Compound 1h-2a:

¹HNMR(400MHz,CDCl₃): δ6.91-6.78(m,2H),4.53 (d,1H),4.46-4.34(m,1H),4.02(d,3H),2.68-2.54 (m,1H),1.93(s,3H),0.88-0.75(m,3H).

### Step ten: Preparation of Compound 1i-2a

1h-2a (1.0 g, 2.7 mmol) was dissolved in tetrahydrofuran (20 mL), and triethylamine (1.64 g, 16.20 mmol) and T3P (3.44 g, 10.79 mmol) were added successively. The mixture was stirred at room temperature for 15 minutes, 1h-2 (0.62 g, 4.07 mmol) was added, and a reaction was allowed to proceed at room temperature for 18 h under a nitrogen atmosphere. A saturated sodium bicarbonate aqueous solution (40 mL) was added, and the mixture was extracted with ethyl acetate (30 mL×3). The organic phases were combined, dried over anhydrous sodium sulfate, filtered, and concentrated to obtain a crude product. The crude product was purified by silica gel column chromatography, affording Compound 1i-2a (0.919 g, yield 67.47%).

### Step eleven: Preparation of Compound 1-2a

1i-2a (0.919 g, 1.82 mmol) was dissolved in a 7 M ammonia-methanol solution (10 mL), and the mixture was reacted at room temperature for 18 h. The reaction system was concentrated to obtain a crude product. The crude product was subjected to silica gel column chromatography, affording Compound 1-2a (0.87 g, yield 97.66%). The absolute configuration of Compound 1-2a was verified by Micro-ED.

### Step twelve: Preparation of Compounds 1-3a and 1-3b

1-2a (0.22 g, 0.45 mmol) was dissolved in dichloromethane (5 mL), and m-chloroperoxybenzoic acid (0.078 g, 0.45 mmol) was added. After the addition was completed, the mixture was reacted at room temperature for 2 h. A saturated sodium bicarbonate solution (10 mL) was added to the reaction system to quench the reaction, and the mixture was extracted with dichloromethane (15 mL×3). The organic phases were combined, dried over anhydrous sodium sulfate, filtered, and concentrated to obtain a crude product. The crude product was purified by silica gel column chromatography, affording Compound 1-3P1 (0.198 g, yield 87.15%) and Compound 1-3P2 (0.018 g, yield 7.92%).

The structure of Compound 1-3P1 was one of Formulas 1-3a and 1-3b above. Moreover, Compound 1-3P1 and Compound 1-3P2 was diastereomers of each other. That is, when the structure of Compound 1-3P1 was the structure of Formula 1-3a, the structure of Compound 1-3P2 was the structure of Formula 1-3b; when the structure of Compound 1-3P1 was the structure of Formula 1-3b, the structure of Compound 1-3P2 was the structure of Formula 1-3a.
Compound 1-3P1: LCMS m/z =506.0 [M+H]⁺
Compound 1-3P2: LCMS m/z =506.1 [M+H]⁺

### Example 2: Preparation of Compounds 6-3 and 6-4

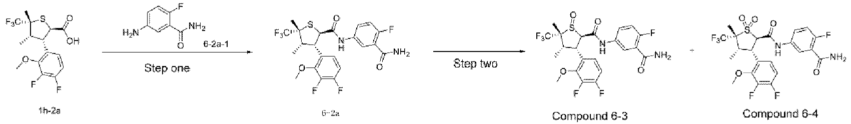

### Step one: Preparation of Compound 6-2a

1h-2a (0.037 g, 0.1 mmol) was dissolved in tetrahydrofuran (3 mL), and triethylamine (0.061 g, 0.60 mmol), T3P (0.26 g, 0.40 mmol), and 6-2a-1 (0.023 g, 0.15 mmol) were added successively. The mixture was reacted at room temperature for 18 h under a nitrogen atmosphere. A saturated sodium bicarbonate aqueous solution (10 mL) was added, and the mixture was extracted with ethyl acetate (10 mL×3). The organic phases were combined, dried over anhydrous sodium sulfate, filtered, and concentrated to obtain a crude product. The crude product was purified by silica gel column chromatography, affording Compound 6-2a (0.036 g, yield 71.08%).

¹HNMR(400MHz,CDCl₃): δ9.69 (s,1H),8.45-8.37 (m,1H),8.10-8.04 (m,1H),7.15-7.06 (m,1H), 6.98 -6.88 (m,2H), 6.87-6.78 (m,1H), 6.47(s,1H),4.84(d,1H), 4.63-4.54 (m,1H),4.08(d,3H), 2.73-2.63(m,1H),1.93(s,3H),0.93-0.83(m,3H).

### Step two: Preparation of Compounds 6-3 and 6-4

6-2a (1.19 g, 2.0 mmol) was dissolved in dichloromethane (10 mL), and m-chloroperoxybenzoic acid (0.41 g, 2.0 mmol) was added. The mixture was reacted at room temperature for 18 h. Saturated sodium bicarbonate (20 mL) was added to the reaction system, and the mixture was extracted with dichloromethane (20 mL×3). The organic phases were combined, dried over anhydrous sodium sulfate, filtered, and concentrated to obtain a crude product. The crude product was purified by silica gel column chromatography, affording Compound 6-3 (0.570 g, yield 54.63%) and Compound 6-4 (0.035 g, yield 3.25%).
Compound 6-3: LCMS m/z =523.3 [M+H]⁺
Compound 6-4: LCMS m/z =539.3 [M+H]⁺

### Example 3: Preparation of Compound 3

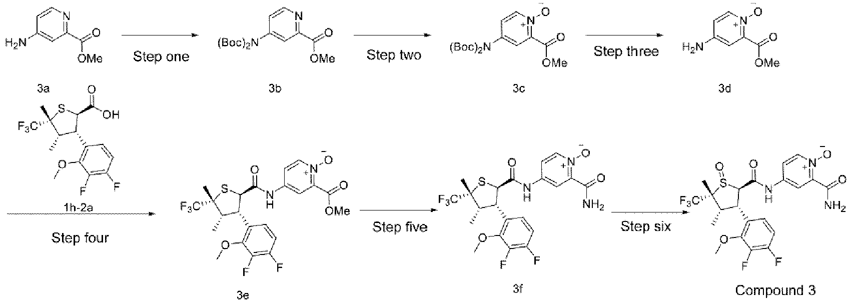

### Step one: Preparation of Compound 3b

3a (3.04 g, 20.0 mmol), (Boc)₂O (4.8 g, 22 mmol), and 4-dimethylaminopyridine (244 mg, 2.0 mmol) were added to dichloromethane (70 mL), and the mixture was reacted at room temperature for 16 h. The reaction solution was concentrated under reduced pressure, followed by being subjected to column chromatography, affording 3b (2.38 g, yield 33.8%).

LCMS m/z =353.4 [M+H]⁺

### Step two: Preparation of Compound 3c

3b (352 mg, 1.0 mmol) was added to dichloromethane (10 mL), and the mixture was placed in an ice bath. m-chloroperoxybenzoic acid (516 mg, 3.0 mmol) was added, and a reaction was allowed to proceed at room temperature for 12 h. The reaction solution was concentrated under reduced pressure, followed by being subjected to isolation and purification by a silica gel column, affording 3b (211 mg, yield 57.3%).

LCMS m/z =369.2 [M+H]⁺

### Step three: Preparation of Compound 3d

3c (134 mg, 0.364 mmol) was added to dichloromethane (2 mL), then a solution of hydrogen chloride in 1,4-dioxane (4 N, 4 mL) was added to the system, and the mixture was reacted at room temperature for 2 h. The reaction solution was concentrated under reduced pressure, then dichloromethane (2 mL) was added, and then a solution of hydrogen chloride in 1,4-dioxane (4 N, 4 mL) was added to the system, and a reaction was allowed to proceed for 2 h. The reaction solution was cooled and concentrated under reduced pressure, affording 3d (71 mg).

LCMS m/z =169.1 [M+H]⁺

### Step four: Preparation of Compound 3e

3d (71 mg) was dissolved in tetrahydrofuran (8 mL), then triethylamine (121.4 mg, 1.2 mmol) and T3P (50%wt in EtOAc 509 mg, containing 1-propylphosphonic anhydride 254.5 mg, 0.8 mmol) were added successively, and finally Substrate 1h-2a (74 mg, 0.2 mmol) was added to the system. After the addition was completed, the mixture was reacted at room temperature for 20 h under a nitrogen atmosphere. A sodium bicarbonate aqueous solution (20 mL) was added to the reaction system to quench the reaction, and the reaction was extracted with ethyl acetate (20 mL×3). The organic phases were combined, dried over anhydrous sodium sulfate, filtered, and concentrated to obtain a crude product. The crude product was separated and purified by a silica gel column, affording 3e (81 mg, yield 77.8%).

LCMS m/z =521.1 [M+H]⁺

### Step five: Preparation of Compound 3f

Substrate 3e (81 mg, 0.155 mmol) was dissolved in a 7 M ammonia-methanol solution (10 mL), and the mixture was reacted at room temperature for 2 h. The reaction system was concentrated to obtain a crude product, and the crude product was subjected to silica gel column chromatography, affording 3f (42 mg, yield 53.6%).

LCMS m/z =506.1 [M+H]⁺

### Step six: Preparation of Compound 3

Substrate 3f (32.0 mg, 0.063 mmol) was dissolved in dichloromethane (3 mL), and m-chloroperoxybenzoic acid (13.0 mg, 0.063 mmol, purity 85%) was added to the system. Then the mixture was reacted at room temperature for 18 h. The reaction was concentrated under reduced pressure. The residue was dispersed in a saturated sodium bicarbonate solution (10 mL), and extracted with ethyl acetate (10 mL×3). The organic phases were combined, and back-washed with a saturated sodium chloride aqueous solution (30 mL). The organic phase was collected, dried over anhydrous sodium sulfate, filtered, and concentrated. The residue was subjected to prep-HPLC preparation and freeze dried, affording Compound 3 (10.0 mg, yield 30.29%). Conditions for prep-HPLC preparation:
instrument: Waters AutoP was used, preparation column: Sunfire C18 (30mm×150mm). Sample-making method: the crude product was dissolved in methanol and filtered with a 0.45 µm filter head to make a sample solution. Mobile phase system: acetonitrile/water (0.1% TFA); gradient elution, acetonitrile content 10% to 60%, flow rate: 30 mL/min, elution time: 25 min.

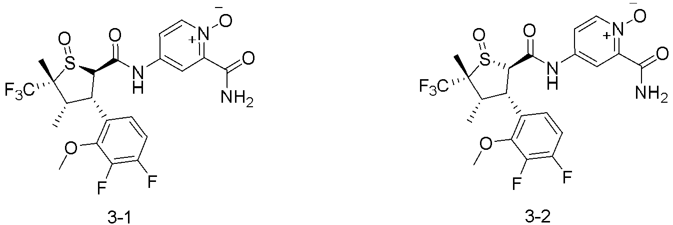

The structure of Compound 3 was one of Formulas 3-1 and 3-2 above.

LCMS m/z = 522.0 [M+H]⁺;

¹HNMR(400MHz, DMSO-*d₆*): δ 11.67 (s, 1H), 10.52 (d, 1H), 8.46 (d, 1H), 8.32 (d, 1H), 8.25 (d, 1H), 7.77 - 7.71 (m, 1H), 7.30 - 7.15 (m, 2H), 4.73 (d, 1H), 4.38-4.30 (m, 1H), 3.98 (d, 3H), 2.94 - 2.83 (m, 1H), 1.62 (s, 3H), 1.09 - 0.98 (m, 3H).

### Example 4: Preparation of Compound 4

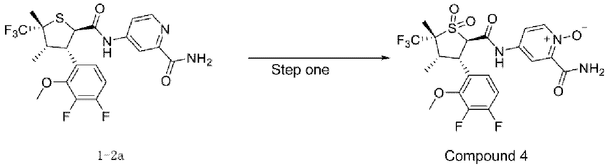

### Step one: Preparation of Compound 4

Substrate 1-2a (50.0 mg, 0.096 mmol) was dissolved in dichloromethane (1 mL), and m-chloroperoxybenzoic acid (88.0 mg, 0.43 mmol, purity 85%) was added to the system. Then the mixture was reacted at room temperature for 18 h. The reaction was concentrated under reduced pressure. The residue was dispersed in a saturated sodium bicarbonate solution (10 mL), and extracted with ethyl acetate (10 mL×3). The organic phases were combined, and back-washed with a saturated sodium chloride aqueous solution (30 mL). The organic phase was collected, dried over anhydrous sodium sulfate, filtered, and concentrated. The residue was subjected to prep-HPLC preparation and freeze dried, affording Compound 4 (36.0 mg, yield 69.86%).

### prep-HPLC preparation conditions:

instrument: Waters AutoP was used, preparation column: Sunfire C18 (30mm×150mm). Sample-making method: the crude product was dissolved in methanol and filtered with a 0.45 µm filter head to make a sample solution. Mobile phase system: acetonitrile/water (0.1% TFA); gradient elution, acetonitrile content 10% to 60%, flow rate: 30 mL/min, elution time: 25 min.

LCMS m/z = 538.0 [M+H]⁺;

¹H NMR (400 MHz, CDCl₃) δ 11.30 (s, 1H), 11.05 (s, 1H), 8.55 - 8.46 (m, 2H), 8.27 (d, 1H), 7.59 (s, 1H), 6.93 - 6.82 (m, 1H), 6.81 - 6.72 (m, 1H), 4.92 (d, 1H), 4.82 - 4.74 (m, 1H), 4.09 (d, 3H), 2.86 - 2.77 (m, 1H), 1.94 (s, 3H), 1.13 - 1.03 (m, 3H).

### Example 5: Preparation of Compound 5

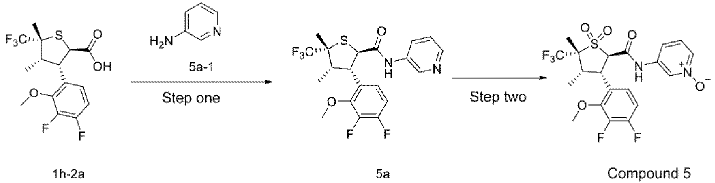

### Step one: Preparation of Intermediate 5a

Substrate 1h-2a (100 mg, 0.27 mmol) was dissolved in a 2 M oxalyl chloride-dichloromethane solution (1 mL), and 0.05 mL DMF was added. The mixture was stirred at room temperature for 2 h. The reaction solution was concentrated under reduced pressure and dissolved in dry dichloromethane (1 mL), and Substrate 5a-1 (27.0 mg, 0.29 mmol) and TEA (79.0 mg, 0.78 mmol) were added successively. The mixture was reacted at room temperature overnight. The reaction solution was concentrated under reduced pressure, and the residue was purified by column chromatography, affording 5a (40 mg, yield 34.83%).

### Step two: Preparation of Compound 5

Substrate 5a (40.0 mg, 0.090 mmol) was dissolved in dichloromethane (1 mL), and m-chloroperoxybenzoic acid (70.0 mg, 0.40 mmol, purity 85%) was added to the system. Then the mixture was reacted at room temperature for 18 h. The reaction was concentrated under reduced pressure. The residue was dispersed in a saturated sodium bicarbonate solution (10 mL), and extracted with ethyl acetate (10 mL×3). The organic phases were combined, and back-washed with a saturated sodium chloride aqueous solution (30 mL). The organic phase was collected, dried over anhydrous sodium sulfate, filtered, and concentrated. The residue was subjected to prep-HPLC preparation and freeze dried, affording Compound 5 (16.0 mg, yield 36.12%).

### Conditions for prep-HPLC preparation:

instrument: Waters AutoP was used, preparation column: Sunfire C18 (30mm×150mm). Sample-making method: the crude product was dissolved in methanol and filtered with a 0.45 µm filter head to make a sample solution. Mobile phase system: acetonitrile/water (0.1% TFA); gradient elution, acetonitrile content 10% to 60%, flow rate: 30 mL/min, elution time: 25 min.

LCMS m/z = 495.1 [M+H]⁺

¹H NMR (400 MHz, CDCl₃) δ 11.31 (s, 1H), 8.76 (s, 1H), 8.31- 8.07 (m, 2H), 7.38 (s, 1H), 7.04 - 6.82 (m, 2H), 5.14 (d, 1H), 4.74 - 4.61 (m, 1H), 4.07 (d, 3H), 2.83 - 2.70 (m, 1H), 1.90 (s, 3H), 1.08 - 0.97 (m, 3H).

### Example 6: Preparation of Compound 6

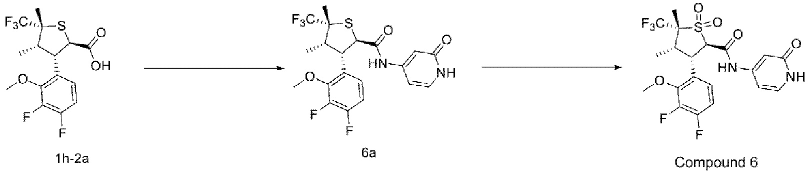

### Step one: Compound 6a was synthesized according to the synthesis method in Example 5.

### Step two: Preparation of Compound 6

Substrate 6a (90 mg, 0.19 mmol) was dissolved in dichloromethane (3 mL), and m-chloroperoxybenzoic acid (330 mg, 1.9 mmol) was added to the system. Then the mixture was reacted at room temperature for 18 h. The reaction was concentrated under reduced pressure. The residue was dispersed in a saturated sodium bicarbonate solution (10 mL), and extracted with ethyl acetate (10 mL×3). The organic phases were combined, and back-washed with a saturated sodium chloride aqueous solution (30 mL). The organic phase was collected, dried over anhydrous sodium sulfate, filtered, and concentrated. The residue was subjected to prep-HPLC preparation and freeze dried, affording Compound 6 (17.0 mg, yield 17.67%).

### Conditions for prep-HPLC preparation:

instrument: waters 2767 preparative liquid phase; chromatography column: xselect CSH Prep C18 (19mm×250mm). The sample was dissolved in DMF and filtered with a 0.45 µ m filter head to make a sample solution. Conditions for preparative chromatography: a. composition of mobile phases A and B: mobile phase A: acetonitrile, mobile phase B: water (containing 0.05 M ammonium bicarbonate); b. gradient elution, mobile phase A content from 30% to 80%; c. flow rate 12 ml/min.; d. elution time 15 min.

LCMS m/z = 495.0 [M+H]⁺;

¹H NMR (400 MHz, DMSO-*d₆*) δ 11.31 (s, 1H), 10.78 (s, 1H), 7.31 (d, 1H), 7.28-7.20 (m, 1H), 6.99-6.92 (m, 1H), 6.57 (d, 1H), 6.27-6.22 (m, 1H), 5.11 (d, 1H), 4.53-4.45 (m, 1H), 4.02 (d, 3H), 2.88-2.79 (m, 1H), 1.82 (s, 3H), 0.97-0.89 (m, 3H).

### Example 7: Preparation of Compound 7

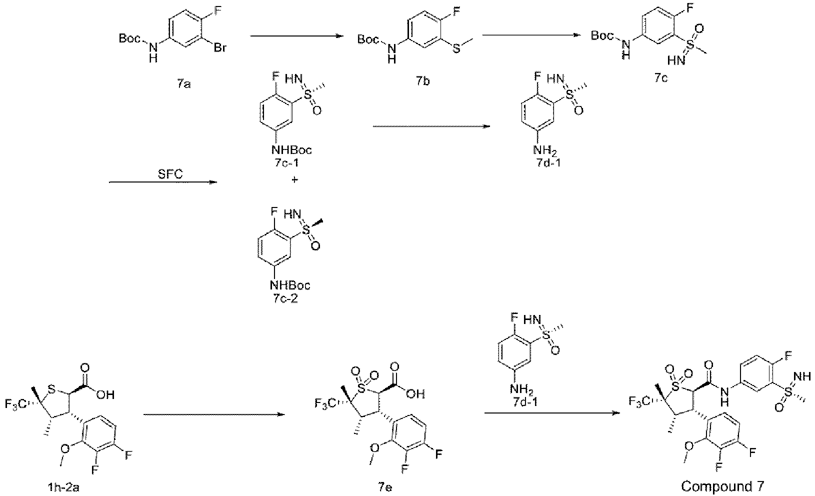

### Step one: Synthesis of Compound 7b

Substrate 7a (6.0 g, 20.7 mmol) was dissolved in dry 1,4-dioxane (65 mL), and then sodium thiomethoxide solid (2.18 g, 31.05 mmol), Pd₂(dba)₃ (1.9 g, 2.07 mmol), Xant Phos (2395.49 mg, 4.14 mmol), and DIPEA (8025.80 mg, 62.10 mmol) were added successively. After the addition was completed, the mixture was heated to 110°C and reacted for 18 h under a nitrogen atmosphere. The reaction system was cooled to room temperature and filtered, and the filter cake was washed with dichloromethane (10 mL×3). The filtrate was collected and concentrated to obtain a crude product. The crude product was purified by silica gel column chromatography, affording the target product 7b (3.4 g, yield 63.83%).

LCMS m/z = 202.0 [M-55]⁺

¹H NMR (400 MHz, CDCl₃): δ 7.40 - 7.34 (m, 1H), 7.08 - 7.01 (m, 1H), 6.94 (t, 1H), 6.40 (s, 1H), 2.47 (s, 3H), 1.52 (s, 9H).

### Step two: Synthesis of Compound 7c

Substrate 7b (3.4 g, 13.21 mmol) was dissolved in methanol (35 mL), and then in an ice bath, ammonium carbamate (1.96 g, 25.10 mmol) and iodobenzene acetate (6.38 g, 19.82 mmol) were added successively. After the addition was completed, the mixture was reacted at room temperature for 18 h. The reaction was concentrated under reduced pressure to obtain a crude product, and the crude product was purified by silica gel column chromatography, affording the target product 7c (2.23 g, yield 58.5%).

LCMS m/z = 289.1 [M+1]⁺

¹H NMR (400 MHz, CDCl₃): δ 7.85 - 7.80 (m, 1H), 7.80 - 7.72 (m, 1H), 7.16 (t, 1H), 6.74 (s, 1H), 3.27 (s, 3H), 1.52 (s, 9H).

### Conditions for SFC preparation:

instrument: SFC Prep 150 AP was used, preparation column: IG (19mm×250mm). Sample-making method: the crude product was dissolved in methanol and filtered with a 0.45 µm filter head to make a sample solution. Mobile phase system: carbon dioxide/methanol (0.05% ammonia water), methanol content 18%; flow rate: 43 mL/min.

Compound 7c (2.23 g) was subjected to SFC preparation and concentrated, affording Compound 7c-1 (1.07 g, retention time: 7.43 min) and Compound 7c-2 (1.12 g, retention time: 11.9 min).

### Step three: Preparation of Compound 7d-1

Compound 7c-1 (430 mg, 1.5 mmol) was dissolved in 1,4-dioxane (2.5 mL), and then a solution of 4 M hydrochloric acid in 1,4-dioxane (2.5 mL) was added dropwise. The mixture was heated to 55°C and reacted for 2 h. The system was concentrated under reduced pressure to obtain 7d-1 (335 mg). The crude product was directly used in the reaction of the next step without any purification.

LCMS m/z = 189.1 [M+H]⁺

### Step four: Preparation of Compound 7e

Substrate 1h-2a (1 g, 2.70 mmol) was dissolved in dichloromethane (20 mL), and m-chloroperoxybenzoic acid (2.33 g, 13.5 mmol) was added to the system. Then the mixture was reacted at room temperature for 18 h. The reaction was concentrated under reduced pressure, and the residue was subjected to prep-HPLC preparation and freeze dried, affording Compound 7e (600 mg, yield 55.23%).

### Step two: Preparation of Compound 7

Substrate 7e (50 mg, 0.12 mmol) was dissolved in a 2 M oxalyl chloride-dichloromethane solution (1 mL), and 0.05 mL DMF was added. The mixture was stirred at room temperature for 2 h. The reaction solution was concentrated under reduced pressure, and dissolved in dry dichloromethane (1 mL). Substrate 7d-1 (33.9 mg, 0.18 mmol) and TEA (60.7 mg, 0.60 mmol) were added successively, and the mixture was reacted at room temperature overnight. The reaction solution was concentrated under reduced pressure, and the residue was purified by column chromatography, affording Compound 7 (10.3 mg, yield 14.48%).

LCMS m/z = 573.0 [M+H]⁺;

¹H NMR (400 MHz, DMSO-*d₆*) δ 11.10 (s, 1H), 8.14-8.09 (m, 1H),7.86-7.80 (m, 1H), 7.40 (t, 1H), 7.27-7.19 (m, 1H), 7.01-6.94 (m, 1H), 5.08 (d, 1H), 4.70 (s, 1H), 4.58-4.50 (m, 1H), 4.03 (d, 3H), 3.16 (s, 3H), 2.90-2.81 (m, 1H), 1.83 (s, 3H), 0.98-0.91 (m, 3H).

### Example 8: Preparation of Compound 8

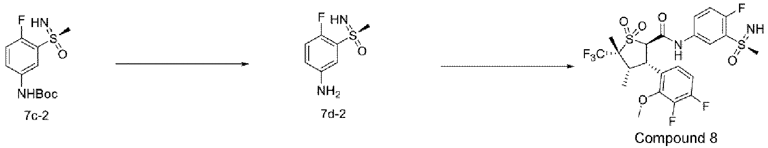

Compound 8 (24 mg, yield 33.73%) was obtained by using 7c-2 as a substrate and referring to the synthesis method of Example 7.

LCMS m/z = 573.2 [M+H]⁺;

### Example 9: Preparation of Compound 9

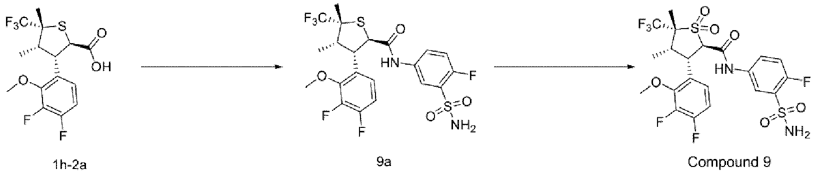

### Step one: Compound 9a was synthesized according to the synthesis method in Example 5.

### Step two: Preparation of Compound 9

Substrate 9a (50.0 mg, 0.092 mmol) was dissolved in dichloromethane (1 mL), and m-chloroperoxybenzoic acid (79.0 mg, 0.46 mmol, purity 85%) was added to the system. Then the mixture was reacted at room temperature for 18 h. The reaction was concentrated under reduced pressure. The residue was dispersed in a saturated sodium bicarbonate solution (10 mL), and extracted with ethyl acetate (10 mL×3). The organic phases were combined, and back-washed with a saturated sodium chloride aqueous solution (30 mL). The organic phase was collected, dried over anhydrous sodium sulfate, filtered, and concentrated. The residue was subjected to prep-HPLC preparation and freeze dried, affording Compound 9 (20.0 mg, yield 37.77%).

### Conditions for prep-HPLC preparation:

instrument: Waters AutoP was used, preparation column: Sunfire C18 (30mm×150mm). Sample-making method: the crude product was dissolved in methanol and filtered with a 0.45 µm filter head to make a sample solution. Mobile phase system: acetonitrile/water (0.1% TFA); gradient elution, acetonitrile content 10% to 60%, flow rate: 30 mL/min, elution time: 25 min.

LCMS m/z = 575.1 [M+H]⁺;

¹H NMR (400 MHz, CDCl₃) δ 9.09 (s, 1H), 8.23 - 8.16 (m, 1H), 7.84 - 7.78 (m, 1H), 7.10 (t, 1H), 6.93 - 6.82 (m, 2H), 5.56 (s, 2H), 4.87 (d, 1H), 4.73 - 4.64 (m, 1H), 4.09 (d, 3H), 2.85 - 2.74 (m, 1H), 1.91 (s, 3H), 1.13 - 1.04 (m, 3H).

### Example 10: Preparation of Compound 10

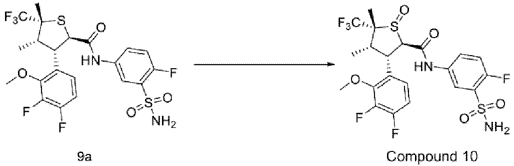

### Step one: Preparation of Compound 10

Substrate 9a (675.0 mg, 1.24 mmol) was dissolved in dichloromethane (3 mL), and m-chloroperbenzoic acid (251.75 mg, 1.24 mmol) was added. After the addition was completed, the mixture was reacted at room temperature for 2 h. The reaction system was concentrated under reduced pressure, and the residue was dissolved in ethyl acetate (20 mL). In an ice bath, a saturated sodium bicarbonate aqueous solution (10 mL) was added, and the mixture was extracted with ethyl acetate (10 mL×3). The organic phases were combined, dried over anhydrous sodium sulfate, filtered, and concentrated to obtain a crude product. The crude product was purified by silica gel column chromatography, affording Compound 10 (567.0 mg, yield 81.59%).

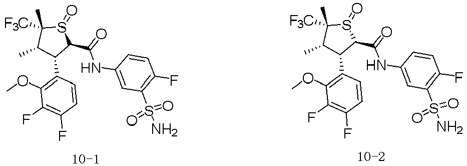

The structure of Compound 10 was one of Formulas 10-1 and 10-2 above.

LCMS m/z = 559.1 [M+H]⁺;

¹H NMR (400 MHz, CDCl₃) δ 9.73 (s, 1H), 8.25 - 8.19 (m, 1H), 7.86 - 7.81 (m, 1H), 7.17 (t, 1H), 7.05 - 6.99 (m, 1H), 6.97 - 6.88 (m, 1H), 6.03 (s, 2H), 4.82 (d, 1H), 4.51 - 4.44 (m, 1H), 4.06 (d, 3H), 2.93 - 2.84 (m, 1H), 1.71 (s, 3H), 1.18 - 1.11 (m, 3H).

### Example 11: Preparation of Compound 11

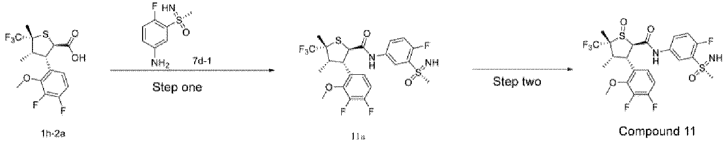

### Step one: Preparation of 11a

In an ice bath, 1h-2a (370.33 mg, 1.0 mmol) was dissolved in DCM (5 mL), and oxalyl chloride (253.86 mg, 2.0 mmol) and DMF (7.31 mg, 0.10 mmol) were added successively. The mixture was stirred in the ice bath for 1 h. The system was concentrated, and the residue was dissolved in DCM (2 mL) to obtain a stock solution. Separately, 7d-1 (282.33 mg, 1.5 mmol) was dissolved in DCM (3 mL), and triethylamine (505.95 mg, 5 mmol) was added. The stock solution was added dropwise to the system. After the addition was completed, the mixture was reacted at room temperature for 18 h under a nitrogen atmosphere. A sodium bicarbonate aqueous solution (20 mL) was added to the reaction system, and the mixture was extracted with ethyl acetate (10 mL×3). The organic phases were combined, dried over anhydrous sodium sulfate, filtered, and concentrated to obtain a crude product. The crude product was purified by silica gel column chromatography, affording Compound 11a (235.0 mg, yield 43.48%).

LCMS m/z = 541.2 [M+H]⁺;

The absolute configuration of Compound 11a was identified by MicroED.

### Step two: Preparation of Compound 11

Compound 11 (70.0 mg, yield 62.89%) was synthesized from Substrate 11a (108.11 mg, 0.2 mmol) by referring to the synthesis method of Example 10.

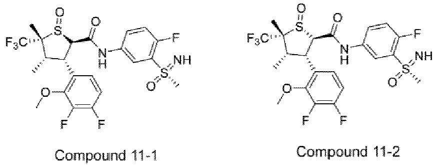

The structure of Compound 11 was one of Formulas 11-1 and 11-2 above.

LCMS m/z = 557.0 [M+H]⁺;

¹H NMR (400 MHz, DMSO-*d₆*) δ 11.36 (s, 1H), 8.19 - 8.14 (m, 1H), 7.92 - 7.85 (m, 1H), 7.42 (t, 1H), 7.32 - 7.20 (m, 2H), 4.88 (d, 1H), 4.72 (s, 1H), 4.39 - 4.32 (m, 1H), 4.00 (d, 3H), 3.18 (s, 3H), 2.94 - 2.85 (m, 1H), 1.65 (s, 3H), 1.11 - 1.02 (m, 3H).

### Example 12: Preparation of Compound 12-1 and Compound 12-2

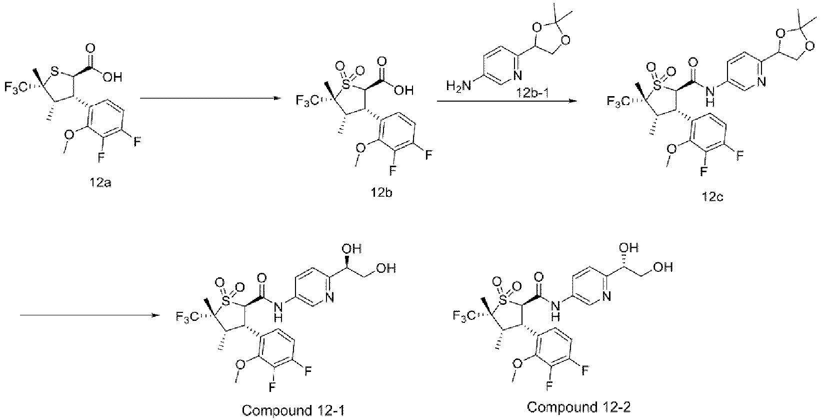

### Step one: Preparation of 12b

Substrate 12a (1 g, 2.70 mmol) was dissolved in dichloromethane (30 mL), and m-chloroperbenzoic acid (2.33 g, 13.5mmol) was added. After the addition was completed, the mixture was reacted at room temperature for 2 h. The reaction system was concentrated under reduced pressure, and the residue was dissolved in ethyl acetate (20 mL). In an ice bath, a saturated sodium bicarbonate aqueous solution (40 mL) was added, and the mixture was extracted with ethyl acetate (10 mL×3). The organic phases were combined, dried over anhydrous sodium sulfate, filtered, and concentrated to obtain a crude product. The crude product was purified by silica gel column chromatography, affording Compound 12b (610.0 mg, yield 56.15%).

### Step two: Preparation of 12c

In an ice bath, 12b (50 mg, 0.12 mmol) was dissolved in DCM (5 mL), and oxalyl chloride (304.63 mg, 2.4 mmol) and DMF (0.05 mL) were added successively. The mixture was stirred in the ice bath for 1 h. The system was concentrated, and the residue was dissolved in DCM (2 mL) to obtain a stock solution. Separately, 12b-1 (34.96 mg, 0.18 mmol) was dissolved in DCM (3 mL), and triethylamine (60.71 mg, 0.60 mmol) was added. The stock solution was added dropwise to the system. After the addition was completed, the mixture was reacted at room temperature for 18 h under a nitrogen atmosphere. A sodium bicarbonate aqueous solution (20 mL) was added to the reaction system, and the mixture was extracted with ethyl acetate (10 mL×3). The organic phases were combined, dried over anhydrous sodium sulfate, filtered, and concentrated to obtain a crude product. The crude product was purified by silica gel column chromatography, affording Compound 12c (40.0 mg, yield 55.63%).

### Step three: Preparation of Compound 12-1 and Compound 12-2

12c (40.0 mg, 0.069 mmol) was dissolved in dichloromethane (3 mL), and trifluoroacetic acid (0.6 mL) was added dropwise. The mixture was reacted at room temperature for 2 h. The reaction system was concentrated to obtain a crude product, and the crude product was purified by silica gel column chromatography (dichloromethane/methanol = 10/1), affording a mixture of Compound 12-1 and Compound 12-2 (15.0 mg, yield 40.29%).

LCMS m/z = 539.2 [M+H]⁺;

### Example 13: Preparation of Compound 13-1 and Compound 13-2

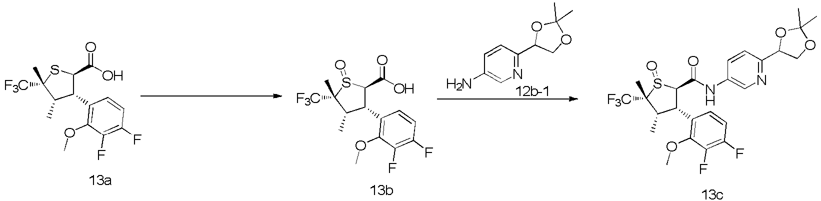

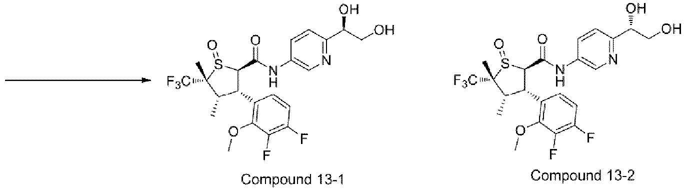

### Step one: Preparation of 13b

Substrate 13a (500 mg, 1.08 mmol) was dissolved in dichloromethane (10 mL), and m-chloroperbenzoic acid (186.38 mg, 1.08 mmol) was added. After the addition was completed, the mixture was reacted at room temperature for 2 h. The reaction system was concentrated under reduced pressure, and the residue was dissolved in ethyl acetate (20 mL). In an ice bath, a saturated sodium bicarbonate aqueous solution (40 mL) was added, and the mixture was extracted with ethyl acetate (10 mL×3). The organic phases were combined, dried over anhydrous sodium sulfate, filtered, and concentrated to obtain a crude product. The crude product was purified by silica gel column chromatography, affording Compound 13b (210.0 mg, yield 40.60%).

### Step two: Preparation of 13c

In an ice bath, 13b (50 mg, 0.13 mmol) was dissolved in DCM (5 mL), and oxalyl chloride (330.02 mg, 2.6 mmol) and DMF (0.05 mL) were added successively. The mixture was stirred in the ice bath for 1 h. The system was concentrated, and the residue was dissolved in DCM (2 mL) to obtain a stock solution. Separately, 12b-1 (37.87 mg, 0.20 mmol) was dissolved in DCM (3 mL), and triethylamine (65.77 mg, 0.65 mmol) was added. The stock solution was added dropwise to the system. After the addition was completed, the mixture was reacted at room temperature for 18 h under a nitrogen atmosphere. A sodium bicarbonate aqueous solution (20 mL) was added to the reaction system, and the mixture was extracted with ethyl acetate (10 mL×3). The organic phases were combined, dried over anhydrous sodium sulfate, filtered, and concentrated to obtain a crude product. The crude product was purified by silica gel column chromatography, affording Compound 13c (11.0 mg, yield 15.11%).

### Step three: Preparation of Compound 13-1 and Compound 13-2

13c (11.0 mg, 0.020 mmol) was dissolved in dichloromethane (2 mL), and trifluoroacetic acid (0.2 mL) was added dropwise. The mixture was reacted at room temperature for 2 h. The reaction system was concentrated to obtain a crude product, and the crude product was purified by silica gel column chromatography (dichloromethane/methanol = 10/1), affording a mixture of Compound 13-1 and Compound 13-2 (3.0 mg, yield 29.36%).

LCMS m/z = 523.2 [M+H]⁺;

### Example 14: Preparation of Compound 14

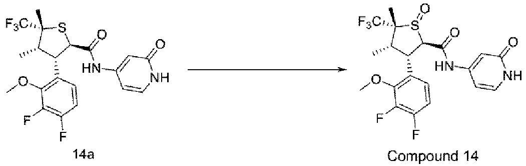

### Step one: Preparation of Compound 14

Substrate 14a (100 mg, 0.22 mmol) (14a was synthesized by referring to WO2024/146632 A1, CAS: 3049923-80-7) was dissolved in dichloromethane (5 mL), and m-chloroperbenzoic acid (37.97 mg, 0.22mmol) was added. After the addition was completed, the mixture was reacted at room temperature for 18 h. The reaction system was concentrated under reduced pressure, and the residue was dissolved in ethyl acetate (20 mL). In an ice bath, a saturated sodium bicarbonate solution (40 mL) was added dropwise, and the mixture was extracted with ethyl acetate (10 mL×3). The organic phases were combined, dried over anhydrous sodium sulfate, filtered, and concentrated to obtain a crude product. The crude product was subjected to prep-HPLC preparation and freeze dried, affording Compound 14 (57.26 mg, yield 55.35%).

### Conditions for prep-HPLC preparation:

instrument: waters 2767 preparative liquid phase was used, preparation column:SUNFIRE@ Prep C18 (19mm×250mm). Sample-making method: the crude product was dissolved in DMF and filtered with a 0.45 µm filter head to make a sample solution. Mobile phase system: acetonitrile/water (containing 50 mM ammonium bicarbonate); gradient elution, acetonitrile content 30% to 60%, flow rate: 15 mL/min, elution time: 14 min.

LCMS m/z = 479.1 [M+H]⁺;

### Example 15: Preparation of Compound 15

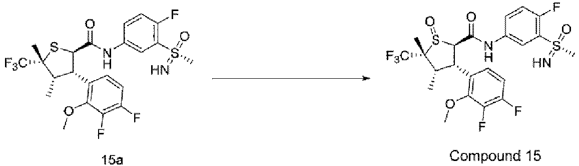

### Step one: Preparation of Compound 15

Substrate 15a (100 mg, 0.19 mmol) (15a was synthesized by referring to WO2024/146632 A1, CAS: 3049925-11-0) was dissolved in dichloromethane (5 mL), and m-chloroperoxybenzoic acid (32.79 mg, 0.19 mmol) was added. After the addition was completed, the mixture was reacted at room temperature for 18 hours. The reaction system was concentrated under reduced pressure, and the residue was dissolved in ethyl acetate (20 mL). In an ice bath, a saturated sodium bicarbonate solution (40 mL) was added dropwise, and the mixture was extracted with ethyl acetate (10 mL×3). The organic phases were combined, dried over anhydrous sodium sulfate, filtered, and concentrated to obtain a crude product. The crude product was subjected to prep-HPLC preparation and freeze dried, affording Compound 15 (33.27 mg, yield 32.31%).

### Conditions for prep-HPLC preparation:

instrument: waters 2767 preparative liquid phase was used, preparation column: SUNFIRE@ Prep C18 (19mm×250mm). Sample-making method: the crude product was dissolved in DMF and filtered with a 0.45 µm filter head to make a sample solution. Mobile phase system: acetonitrile/water (containing 50 mM ammonium bicarbonate); gradient elution, acetonitrile content 30% to 65%, flow rate: 15 mL/min, elution time: 15 min.

LCMS m/z = 556.9 [M+H]⁺.

### Biological Test Example 1

### Nav1.8 Manual Patch Clamp Test

### Culture of cells

A CHO cell line stably expressing human Nav1.8 was cultured in Ham's F-12 medium containing 10% fetal bovine serum and 10 µg/mL Blasticidin, 200 µg/mL Hygromycin B, and 100 µg/mL Zeocin. The cell culture temperature was 37°C, and the carbon dioxide concentration was 5%. The old medium was removed and the cells were rinsed with PBS once. Then 1 mL of a 0.25%-Trypsin-EDTA solution was added, and the cells were incubated at 37°C for about 1.5 min. When the cells detached from the bottom of the dish, complete medium preheated at 37°C was added. The cell suspension was gently pipetted to separate the aggregated cells. The cell suspension was transferred to a sterile centrifuge tube and centrifuged at 1000 rpm for 5 min to collect cells. The cells were seeded in 6-cm cell culture dishes for expansion or maintenance culture, the number of cells seeded in each cell culture dish being 2.5×10 ⁵ cells (the final volume being 5 mL). To maintain the electrophysiological activity of the cells, the cell density could not exceed 80%. Before patch clamp detection, the cells were separated using 0.25%-Trypsin-EDTA. 6.5×10³ cells were plated onto a coverslip, and cultured in a 24-well plate (the final volume being 500 µL) for 18 h for later detection.

### Formulation of compounds

Compounds were dissolved in dimethyl sulfoxide (DMSO) and formulated as 30 mM DMSO stock solutions. The stock solutions were diluted with an extracellular fluid (140 mM NaCl, 3.5 mM KCI, 1 mM MgCl₂•6H₂O, 2 mM CaCl₂•2H₂O, 10 mM D-Glucose, 10 mM HEPES, and 1.25 mM NaH₂PO₄•2H₂O, adjusted to pH 7.4 with NaOH) to test concentrations, and the final concentration of DMSO in all test samples was 0.1%.

### Electrophysiological test

First, a capillary glass tube was pulled with a microelectrode puller to form a recording electrode, and then the electrode filled with an intracellular fluid solution (50 mM CsCl, 10 mM NaCl, 10 mM HEPES, 60 mM CsF, and 20 mM EGTA, adjusted to pH 7.2 with CsOH) was placed in a microelectrode holder. A microelectrode manipulator was operated under an inverted microscope to immerse the electrode in the extracellular fluid, and the resistance (Rpip) of the electrode was recorded. Then the electrode was slowly brought into contact with a cell surface, and negative pressure suction was applied to form a GΩ seal. At this time, fast capacitance compensation was performed, and application of negative pressure suction was continued to break the cell membrane to form a whole-cell recording mode. Finally, slow capacitance compensation was performed, and experimental parameters such as series resistance (Rs) were recorded. No leakage compensation was given. Drug administration was started when the Nav1.8 current recorded in the whole-cell recording mode became stable. For each drug concentration, drug action was allowed for about 5 min (or until the current became stable). The coverslip plated with the cells was placed in a recording bath under the inverted microscope. The extracellular fluid as a blank control and the working solution of the test compound were allowed to flow through the recording bath by means of gravity perfusion to act on the cells. Liquid exchange was performed using a peristaltic pump. The current detected in the cells in the compound-free extracellular fluid was used as a control group. All electrophysiological tests were performed at room temperature. The inhibition rate of the compound on Nav1.8 was determined by calculating the relative percentage of the peak currents generated before and after treatment of the cells with the compound.

The voltage stimulation protocol for whole-cell patch clamp recording of Nav1.8 sodium current was as follows: The cell voltage was clamped at - 120 mV upon formation of a whole-cell seal. The voltage was first stepped from -110 mV to -30 mV in 10-mV steps. After maintaining for 5 s, a depolarization pulse to 0 mV was applied to obtain a half-inactivation voltage (V_{half}). Subsequently, the V_{half} was used as a stimulation voltage and maintained for 5 s, and then the voltage was restored to -120 mV and maintained for 20 ms. A further depolarization pulse (TP2) was applied to 0 mV for 50 ms to detect the sodium current in the half-inactivated state. Finally, the clamp voltage was restored to -120 mV. Data were repeatedly collected every 20 ms to observe the effect of the drug on the sodium current peak. The test data were collected by an EPC10 amplifier (HEKA) and stored in PatchMaster (HEKA) software.

**Table 1. Inhibitory activity (IC₅₀) of test compounds against Nav1.8**

| Test compound | IC₅₀ (nM) |
|---|---|
| Compound 1-3P1 | <1 |
| Compound 1-3P2 | <1 |
| Compound 3 | <1 |
| Compound 4 | <0.1 |
| Compound 5 | <1 |
| Compound 6 | <1 |
| Compound 6-3 | <0.1 |
| Compound 6-4 | <0.1 |
| Compound 9 | <0.1 |
| Compound 10 | <1 |

Conclusion: The compounds of the present invention, e.g., the compounds of the examples, had a good inhibitory activity against Nav1.8. Specifically, for example, compound 4 had an IC₅₀ of 0.016 nM, and compound 9 had an IC₅₀ of 0.096 nM.

### Biological Test Example 2: Pharmacokinetic Test in Rats

Test animals: Male SD rats, about 220 g, six to eight weeks old, six rats for each compound.

Experimental design: On the day of the test, twenty-four SD rats were randomly grouped according to body weight. The rats were subjected to fasting fasting with free access to water for 12 h to 14 h one day before administration, and were fed 4 h after administration.

**Table 2. Administration information**

| Administration dose (mg/kg) | Administration concentration (mg/mL) | Administration volume (mL/kg) | Collected sample | Administration mode |
|---|---|---|---|---|
| 2.5 | 0.5 | 5 | Plasma | Intravenous administration |
| 10 | 1 | 10 | Plasma | Intragastric administration |

| | | | | |
|---|---|---|---|---|
| Note: Vehicle for intravenous administration: 5% DMA + 5% Solutol + 90% Saline; Vehicle for intragastric administration: 0.5% MC (DMA: dimethylacetamide; Solutol: polyethylene glycol-15-hydroxystearate; Saline: physiological saline; MC: methylcellulose) | | | | |

Before and after administration, 0.10 mL of blood was collected from the eye orbit under isoflurane anesthesia, placed in an EDTAK2 centrifuge tube, centrifuged at 5000 rpm, 4°C for 10 min, and the plasma was collected. The blood collection time points for both the intravenous administration group and the intragastric administration were: 0 min, 5 min, 15 min, 30 min, 1 h, 2 h, 4 h, 6 h, 8 h, and 24 h. Prior to analytical detection, all samples were stored at -80°C. The samples were quantitatively analyzed by LC-MS/MS.

Conclusion: The compounds of the present invention, e.g., the compounds of the examples, had good oral properties in rats.

### Biological Test Example 3: Pharmacokinetic Test in Mice

Test animals: Male C57 mice, 22 g to 25 g, six mice for each compound.

Experimental design: On the day of the test, the C57 mice were randomly grouped according to body weight. The mice were subjected to fasting with free access to water for 12 h to 14 h one day before administration, and were fed 4 h after administration.

**Table 3. Administration information**

| Administration dose (mg/kg) | Administration concentration (mg/mL) | Administration volume (mL/kg) | Collected sample | Administration mode |
|---|---|---|---|---|
| 2.5 | 0.5 | 5 | Plasma | Intravenous administration |
| 30 | 3 | 10 | Plasma | Intragastric administration |

| | | | | |
|---|---|---|---|---|
| Note: Vehicle for intravenous administration: 5% DMA + 5% Solutol + 90% Saline; Vehicle for intragastric administration: 0.5% MC (DMA: dimethylacetamide; Solutol: polyethylene glycol-15-hydroxystearate; Saline: physiological saline; MC: methylcellulose) | | | | |

Before and after administration, 0.06 mL of blood was collected from the eye orbit under isoflurane anesthesia, placed in an EDTAK2 centrifuge tube, centrifuged at 5000 rpm, 4°C for 10 min, and the plasma was collected. The blood collection time points for both the intravenous administration group and the intragastric administration were: 0 min, 5 min, 15 min, 30 min, 1 h, 2 h, 4 h, 7 h, 24 h, and 48 h. Prior to analytical detection, all samples were stored at -80°C. The samples were quantitatively analyzed by LC-MS/MS.

**Table 3-1. Pharmacokinetic results of test compounds in mice**

| Test compound | Administration dose | AUC₀₋ₜ (hr×ng/mL) | T_{1/2} |
|---|---|---|---|
| Compound 4 | Intragastric administration 30 mg/kg | 27377 | 4.84 |
| 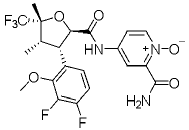 Control compound 1 | Intragastric administration 30 mg/kg | 1471 | 2.08 |

Conclusion: The compounds of the present invention, e.g., the compounds of the examples, had good oral properties in mice.

### Biological Test Example 4: Pharmacokinetic Test in Beagle Dogs

Test animals: Male beagle dogs, about 8 kg to 11 kg, six beagle dogs for each compound, purchased from Beijing Marshall Biotechnology Co.,Ltd.

Test protocol: On the day of the test, twelve beagle dogs were randomly grouped according to body weight. The beagle dogs were subjected to fasting with free access to water for 12 h to 14 h one day before administration, and were fed 4 h after administration. Administration was performed according to Table 4.

**Table 4. Administration information**

| Administration dose (mg/kg) | Administration concentration (mg/mL) | Administration volume (mL/kg) | Collected sample | Administration mode |
|---|---|---|---|---|
| 1 | 1 | 1 | Plasma | Intravenous administration |
| 5 | 1 | 5 | Plasma | Intragastric administration |

| | | | | |
|---|---|---|---|---|
| Note: Intravenous Vehicle: 5% DMA + 5% Solutol + 90% Saline; Vehicle for intragastric administration: 0.5% MC (DMA: dimethylacetamide; Solutol: polyethylene glycol-15-hydroxystearate; Saline: physiological saline; MC: methylcellulose solution;) | | | | |

Before and after administration, 1 ml of blood was collected from the jugular vein or limb vein and placed in an EDTAK2 centrifuge tube. The blood was centrifuged at 5000 rpm, 4°C for 10 min, and the plasma was collected. The blood collection time points for both the intravenous administration group and the intragastric administration were: 0 min, 5 min, 15 min, 30 min, 1 h, 2 h, 4 h, 6 h, 8 h, 10 h, 12 h, 24 h, 48 h, and 72 h. Prior to analytical detection, all samples were stored at -80°C. The samples were quantitatively analyzed by LC-MS/MS.

**Table 4-1. Pharmacokinetic results of test compounds in beagle dogs**

| Test compound | Administration dose | AUC₀₋ₜ (hr×ng/mL) | T_{1/2} |
|---|---|---|---|
| Compound 4 | Intragastric administration 5 mg/kg | 98184 | 93.4 |

Conclusion: The compounds of the present invention, e.g., the compounds of the examples, had good oral properties in beagle dogs.

### Biological Test Example 5: Pharmacokinetic Test in Monkeys

Test animals: Male cynomolgus monkeys, 3 kg to 5 kg, three to six years old, six monkeys for each compound. Purchased from Suzhou Xishan Biotechnology Co., Ltd.

Test protocol: On the day of the test, six monkeys were randomly grouped according to body weight. The monkeys were subjected to fasting with free access to water for 14 h to 18 h one day before administration, and were fed 4 h after administration.

**Table 5. Administration information**

| Administration dose (mg/kg) | Administration concentration (mg/mL) | Administration volume (mL/kg) | Collected sample | Administration mode |
|---|---|---|---|---|
| 1 | 0.5 | 2 | Plasma | Intravenous administration |
| 5 | 1 | 5 | Plasma | Intragastric administration |

| | | | | |
|---|---|---|---|---|
| Note: Intravenous Vehicle: 5% DMA + 5% Solutol + 90% Saline; Vehicle for intragastric administration: 0.5% MC (containing 0.5% Tween 80); *Doses were on a free base basis. | | | | |

Before and after administration, 1.0 mL of blood was collected from the limb vein and placed in an EDTAK2 centrifuge tube. The blood was centrifuged at 5000 rpm, 4°C for 10 min, and the plasma was collected. The blood collection time points for both the intravenous administration group and the intragastric administration group were: 0 min, 5 min, 15 min, 30 min, 1 h, 2 h, 4 h, 6 h, 8 h, 10 h, 12 h, and 24 h. Prior to analytical detection, all samples were stored at -80°C. The samples were quantitatively analyzed by LC-MS/MS.

Conclusion: The compounds of the present invention, e.g., the compounds of the examples, had good oral properties in monkeys.

### Biological Test Example 6: CYP450 Enzyme Inhibition Assay

The purpose of this study was to evaluate, by using an in vitro test system, the effect of a test substance on the activity of five isoenzymes (CYP1A2, CYP2C9, CYP2C19, CYP2D6, and CYP3A4) of human liver microsomal cytochrome P450 (CYP). The specific probe substrates of the CYP450 isozymes were respectively co-incubated with human liver microsomes and different concentrations of the test substance. Reduced nicotinamide adenine dinucleotide phosphate (NADPH) was added to initiate the reaction. After the reaction was completed, the sample was treated, and the metabolite produced by the specific substrate was quantitatively detected using the liquid chromatography-tandem mass spectrometry (LC-MS/MS) method. The change in the activity of the CYP enzyme was determined, the IC₅₀ value was calculated, and the inhibitory potential of the test substance on each CYP enzyme subtype was evaluated.

Conclusion: The compounds of the present invention, e.g., the compounds of the examples, showed weak CYP inhibition.

### Biological Test Example 7: Caco2 Permeability Test

The assay used a monolayer of Caco-2 cells incubated in triplicate in a 96-well Transwell plate. A transport buffer solution (HBSS, 10 mM HEPES, pH 7.4 ± 0.05) containing the compound of the present invention (2 µM) or the control compound digoxin (10 µM), nadolol (2 µM), or metoprolol (2 µM) was added to the donor compartment at the apical side or basolateral side. A transport buffer solution containing DMSO was added to the corresponding receiver compartment. After incubation at 37±1°C for 2 h, the cell plate was removed. An appropriate amount of sample was taken from the apical side and the basolateral side respectively and transferred to a new 96-well plate. Then, acetonitrile containing an internal standard was added for protein precipitation. The samples were analyzed using LC MS/MS, and the concentrations of the compounds of the present invention and the control compounds were determined. The concentration data were used to calculate the apparent permeability coefficients for the apical-to-basolateral transport and the basolateral-to-apical transport across the cell monolayer, and thus to calculate the efflux ratio. The intactness of the cell monolayer after 2 hours of incubation was assessed by leakage of fluorescein.

Conclusion: The compounds of the present invention, e.g., the compounds of the examples, had good permeability.

### Biological Test Example 8: Spinal Nerve Ligation (SNL)-Induced Mouse Neuropathic Pain Model

Male C57BL/6J mice purchased from Zhejiang Vital River Laboratory Animal Technology Co., Ltd. were adaptively fed for one week prior to model construction. The specific construction method was as follows:
(1) the surgical instrument and the ligature were sterilized;
(2) the mice were anesthetized with isoflurane and placed on an operating table in a prone position;
(3) hair near the hip bone of the mouse was shaved for skin preparation, and an incision of about 2 cm was made along the spine;
(4) the fascia was separated along the spine, and the muscle was bluntly separated to expose the L5 transverse process;
(5) the L5 transverse process was carefully pinched off with forceps to expose the L5 spinal nerve;
(6) the L5 nerve was carefully separated with a glass dissecting needle, and the L5 nerve was ligated using a 5-0 ligature; and
(7) the muscle and skin was sutured and disinfected with iodophor.

The next day after model construction, mice that failed the model construction were eliminated (indicator of successful mouse modeling: hindpaw flexion). After the model construction, the mice were stroked daily for 3 to 5 minutes to ensure the familiarity of the animals with the experimenter. Then the mice were placed on a metal pain-measuring rack for adaptation for 40 to 60 minutes. After completion of environmental adaptation on day 3, the pre-administration baseline value (Ascending test method) of the animals was determined using Von Frey filaments (Aesthesio^{®}; 0.16 g, 0.4 g, 0.6 g, 1.0 g, 1.4 g, and 2.0 g). For each animal, the average value of two measurements, which were made at an interval of at least 5 minutes, was taken. The animals were grouped according to the baseline value (10 animals per group). After grouping, the test compound (3 mg/kg and 30 mg/kg) or vehicle (0.5% methylcellulose) was administered intragastrically, and the mechanical pain threshold (MPT) of the mice was determined at different time points after administration. A Time-MPT curve was plotted with GraphPad 8.3.0, and statistical analysis was performed.

Conclusion: Based on the analysis of the area under the Time-MPT curve, the compounds of the present invention, e.g., the compounds of the Examples, had a significant analgesic effect.

### Biological Test Example 9: Stability Assay in Monkey Liver Microsomes

At 37°C, 1 µM of a test compound was incubated with monkey liver microsomes (0.5 mg/mL) supplemented with a NADPH regeneration system for 5, 15, 30, 45, and 60 minutes. The concentration of the test compound in a resulting sample was detected using the LC-MS/MS method. The half life (T_{1/2}) and intrinsic clearance (CL_{int(mic)}) of the compound in the monkey liver microsome solution were obtained by performing calculation on the remaining percentages of the compound versus the respective corresponding time points.

### Biological test results:

| Compound | T_{1/2} (min) | CL_{int(mic)} (µL/min/mg) |
|---|---|---|
| Compound 4 | > 186 | <7.45 |
| 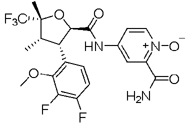 Control compound 1 | 31.1 | 44.5 |

Conclusion: The compounds of the present invention had good stability in monkey liver microsomes. Among the compounds, Compound 4 had a significantly slower clearance rate in monkey liver microsomes and a significantly longer half-life, compared with the control compound. The ratio of the T_{1/2} of Compound 4 to that of Control compound 1 was about 6.

## Claims

1. A compound or a stereoisomer, a deuterate, a solvate, a prodrug, a metabolite, a pharmaceutically acceptable salt, or a cocrystal thereof, the compound being selected from a compound shown in general formula (I), 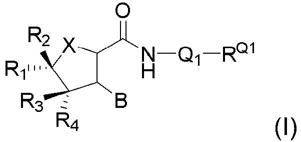
where X is selected from -S(O)- or -S(O)₂-;
Q₁ is selected from C₆₋₁₀ aryl, 5- to 10-membered heteroaryl, C₅₋₁₀ carbocycle, 5- to 10-membered heterocycle, or 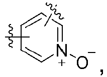 the aryl, heteroaryl, carbocycle, or heterocycle being optionally substituted with 1 to 5 R^{q}'s;
R^{Q1} is selected from H, NR^{q1}R^{q2}, -C(=O)NR^{q1}R^{q2}, -S(=O)₂NR^{q1}R^{q2}, OH, =O, -OR^{q1}, -C(=O)R^{q1}, -S(=O)₂R^{q1}, -S(=O)(=NR^{q1})R^{q2}, or -P(=O)R^{q1}R^{q2};
R^{q1} and R^{q2} are each independently selected from H, C₁₋₆ alkyl, C₃₋₆ carbocycle, or 4- to 7-membered heterocycle, the alkyl, carbocycle, or heterocycle being optionally substituted with 1 to 4 R^{k}'s;
alternatively, R^{q1} and R^{q2} are directly connected to form a 4- to 7-membered heterocycle, the heterocycle being optionally substituted with 1 to ₄ R^{k}'s;
B is selected from C₆₋₁₀ aryl, 5- to 10-membered heteroaryl, C₅₋₁₀ carbocycle, or 5- to 10-membered heterocycle, the aryl, heteroaryl, carbocycle, or heterocycle being optionally substituted with 1 to 5 R^{B}'s;
R¹, R², R³, and R⁴ are each independently selected from H, halogen, CN, OH, C₁₋₆ alkyl, C₂₋₆ alkenyl, C₂₋₆ alkynyl, -OC₁₋₆ alkyl, -SC₁₋₆ alkyl, C₃₋₆ carbocycle, or 3- to 7-membered heterocycle, the alkyl, alkenyl, alkynyl, carbocycle, or heterocycle being optionally substituted with 1 to 4 R^{k}'s;
alternatively, R¹ and R⁴ are directly connected to form a C₃₋₆ carbocycle or 4- to 7-membered heterocycle, the carbocycle or heterocycle being optionally substituted with 1 to 6 R^{k}'s;
R^{q} and R^{B} are each independently selected from H, deuterium, =O, halogen, CN, OH, NH₂, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, -SC₁₋₆ alkyl, C₃₋₇ carbocycle, -OC₃₋₇ carbocycle, 3- to 7-membered heterocycle, or -P(=O)R^{q1}R^{q2}, the alkyl, alkenyl, alkynyl, carbocycle, or heterocycle being optionally substituted with 1 to 4 R^{k}'s; and
R^{k} in each occurrence is independently selected from deuterium, =O, halogen, CN, OH, NH₂, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, -SC₁₋₆ alkyl, -O-C₃₋₆ carbocycle, -O-3- to 7-membered heterocycle, -NH-C₃₋₆ carbocycle, -NH-3- to 7-membered heterocycle, -C₁₋₄ alkylene-C₃₋₆ carbocycle, -C₁₋₄ alkylene-3- to 7-membered heterocycle, C₃₋₆ carbocycle, or 3- to 7-membered heterocycle, the alkyl, alkylene, alkenyl, alkynyl, carbocycle, or heterocycle being optionally substituted with 1 to 4 substituents selected from deuterium, halogen, =O, CN, OH, NH₂, C₁₋₆ alkyl, or C₁₋₆ alkoxy.

2. The compound or a stereoisomer, a deuterate, a solvate, a prodrug, a metabolite, a pharmaceutically acceptable salt, or a cocrystal thereof according to claim 1, wherein
R^{q1} and R^{q2} are each independently selected from H, C₁₋₄ alkyl, C₃₋₆ carbocycle, or 4- to 7-membered heterocycle, the alkyl, carbocycle, or heterocycle being optionally substituted with 1 to 4 R^{k}'s;
alternatively, R^{q1} and R^{q2} are directly connected to form a 4- to 7-membered heterocycle, the heterocycle being optionally substituted with 1 to 4 R^{k}'s;
Q₁ is selected from phenyl, benzo C₄₋₆ carbocycle, benzo 4- to 6-membered heterocycle, 5- to 6-membered heteroaryl, 5- to 6-membered heterocycle, 8- to 10-membered fused heteroaryl, or 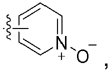 Q₁ being optionally substituted with 1 to 4 R^{q}'s;
B is selected from phenyl, benzo C₄₋₆ carbocycle, benzo 4- to 6-membered heterocycle, 5- to 6-membered heteroaryl, or 8- to 10-membered fused heteroaryl, B being optionally substituted with 1 to 4 R^{B}'s;
R¹, R², R³, and R⁴ are each independently selected from H, halogen, CN, OH, C₁₋₄ alkyl, C₂₋₄ alkenyl, C₂₋₄ alkynyl, -OC₁₋₄ alkyl, -SC₁₋₄ alkyl, C₃₋₆ carbocycle, or 3- to 7-membered heterocycle, the alkyl, alkenyl, alkynyl, carbocycle, or heterocycle being optionally substituted with 1 to 4 R^{k}'s;
alternatively, R¹ and R⁴ are directly connected to form a C₃₋₆ carbocycle or 4- to 7-membered heterocycle, the carbocycle or heterocycle being optionally substituted with 1 to 4 R^{k}'s;
R^{q} and R^{B} are each independently selected from H, deuterium, =O, halogen, CN, OH, NH₂, NHC₁₋₄ alkyl, N(C₁₋₄ alkyl)₂, C₁₋₄ alkyl, C₂₋₄ alkenyl, C₂₋₄ alkynyl, -OC₁₋₄ alkyl, -SC₁₋₄ alkyl, C₃₋₇ carbocycle, -OC₃₋₇ carbocycle, 3- to 7-membered heterocycle, or -P(=O)R^{q1}R^{q2}, the alkyl, alkenyl, alkynyl, carbocycle, or heterocycle being optionally substituted with 1 to 4 R^{k}'s; and
R^{k} in each occurrence is independently selected from deuterium, =O, halogen, CN, OH, NH₂, NHC₁₋₄ alkyl, N(C₁₋₄ alkyl)₂, C₁₋₄ alkyl, C₂₋₄ alkenyl, C₂₋₄ alkynyl, -OC₁₋₄ alkyl, -SC₁₋₄ alkyl, -O-C₃₋₆ carbocycle, or -O-3- to 7-membered heterocycle, the alkyl, alkenyl, alkynyl, carbocycle, or heterocycle being optionally substituted with 1 to 4 substituents selected from deuterium, halogen, CN, OH, NH₂, C₁₋₄ alkyl, or C₁₋₄ alkoxy.

3. The compound or a stereoisomer, a deuterate, a solvate, a prodrug, a metabolite, a pharmaceutically acceptable salt, or a cocrystal thereof according to claim 2, wherein
R^{q1} and R^{q2} are each independently selected from H, methyl, ethyl, propyl, isopropyl, cyclopropyl, cyclobutyl, or cyclopentyl, the methyl, ethyl, propyl, isopropyl, cyclopropyl, cyclobutyl, or cyclopentyl being optionally substituted with 1 to 4 R^{k}'s;
R¹, R², R³, and R⁴ are each independently selected from H, F, Cl, Br, cyano, methyl, ethyl, methoxy, ethoxy, methylthio, cyclopropyl, cyclobutyl, ethenyl, or ethynyl, the methyl, ethyl, methoxy, ethoxy, methylthio, cyclopropyl, cyclobutyl, ethenyl, or ethynyl being optionally substituted with 1 to 4 R^{k}'s;
alternatively, R¹ and R⁴ are directly connected to form a C₃₋₆ carbocycle, the carbocycle being optionally substituted with 1 to 4 R^{k}'s;
B is selected from 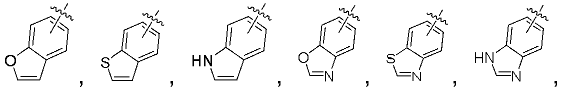 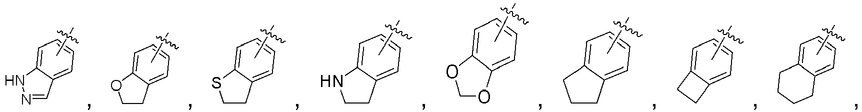 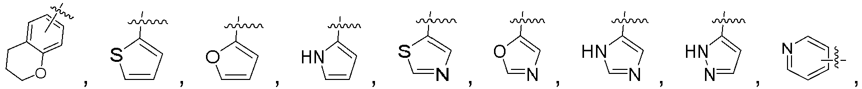 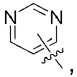 or phenyl, B being optionally substituted with 1 to 4 R^{B}'s;
R^{q} and R^{B} are each independently selected from H, deuterium, =O, F, Cl, Br, cyano, methyl, ethyl, isopropyl, methoxy, ethoxy, isopropyloxy, methylthio, cyclopropyl, cyclopropyl-O-, cyclobutyl, ethenyl, ethynyl,-P(=O)(CH₃)₂, -P(=O)(CH₂CH₃)₂, or -P(=O)(CH₃)(cyclopropyl), the methyl, ethyl, isopropyl, methoxy, ethoxy, isopropyloxy, methylthio, cyclopropyl, cyclobutyl, ethenyl, or ethynyl being optionally substituted with 1 to 4 R^{k}'s; and
R^{k} in each occurrence is independently selected from deuterium, =O, F, Cl, Br, I, CN, OH, NH₂, NH(CH₃), NH(CH₂CH₃), N(CH₃)₂, N(CH₂CH₃)₂, methyl, ethyl, ethenyl, ethynyl, methoxy, ethoxy, methylthio, -O-cyclopropyl, -NH-cyclopropyl, -CH₂-cyclopropyl, -CH₂-cyclobutyl, -CH₂-cyclopentyl, -CH₂-cyclohexyl, cyclopropyl, cyclobutyl, cyclopentyl, or cyclohexyl, the methyl, ethyl, ethenyl, ethynyl, methoxy, ethoxy, methylthio, cyclopropyl, cyclobutyl, cyclopentyl, or cyclohexyl being optionally substituted with 1 to 4 substituents selected from deuterium, halogen, CN, OH, NH₂, C₁₋₄ alkyl, or C₁₋₄ alkoxy.

4. The compound or a stereoisomer, a deuterate, a solvate, a prodrug, a metabolite, a pharmaceutically acceptable salt, or a cocrystal thereof according to claim 3, wherein
Q₁ is selected from 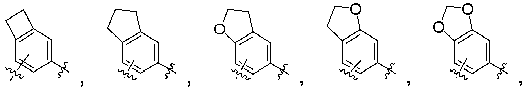 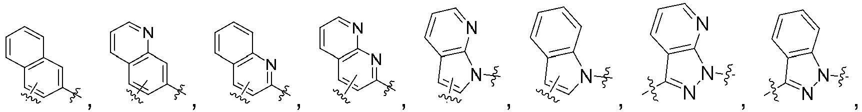 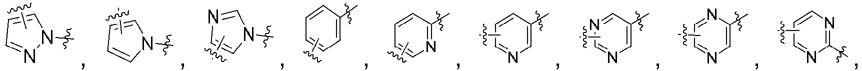 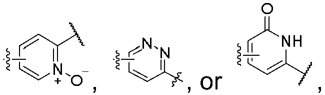 Q₁ being optionally substituted with 1 to 4 R^{q}'s;
R¹, R², R³, and R⁴ are each independently selected from H, methyl, ethyl, CH₂F, CHF₂, or CF₃;
R^{q} and R^{B} are each independently selected from H, =O, F, Cl, Br, cyano, CH₂F, CHF₂, CF₃, -OCH₂F, -OCHF₂, -OCF₃, -OCD₃, methyl, -S-methyl, -S-CF₃, ethyl, isopropyl, ethynyl, methoxy, ethoxy, isopropyloxy, propyloxy, cyclopropyl, -O-cyclopropyl, -P(=O)(CH₃)₂, -P(=O)(CH₂CH₃)₂, or -P(=O)(CH₃)(cyclopropyl), the methyl, ethyl, isopropyl, ethynyl, methoxy, ethoxy, isopropyloxy, propyloxy, or cyclopropyl being optionally substituted with 1 to 4 R^{k}'s; and
R^{k} in each occurrence is independently selected from deuterium, F, Cl, Br, I, CN, OH, -CH₂OH, methyl, ethyl, ethenyl, ethynyl, methoxy, ethoxy, methylthio, -O-cyclopropyl, -NH-cyclopropyl, -CH₂-cyclopropyl, -CH₂-cyclobutyl, -CH₂-cyclopentyl, -CH₂-cyclohexyl, cyclopropyl, cyclobutyl, cyclopentyl, or cyclohexyl.

5. The compound or a stereoisomer, a deuterate, a solvate, a prodrug, a metabolite, a pharmaceutically acceptable salt, or a cocrystal thereof according to claim 4, wherein 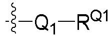 is selected from 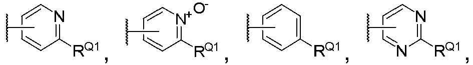 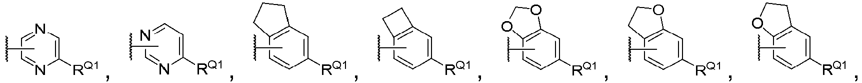 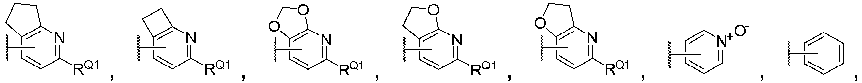 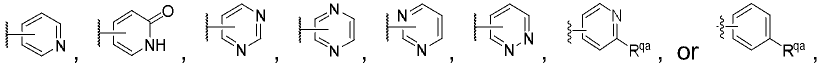 Q₁ being optionally substituted with 1 to 3 R^{q}'s; and
R^{qa} is selected from 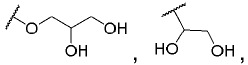 -CH₂OH, -CF₂CH₂OH, NH₂, -P(=O)(CH₃)₂, -P(=O)(CH₂CH₃)₂, or -P(=O)(CH₃)(cyclopropyl).

6. The compound or a stereoisomer, a deuterate, a solvate, a prodrug, a metabolite, a pharmaceutically acceptable salt, or a cocrystal thereof according to claim 5, wherein 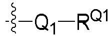 is selected from 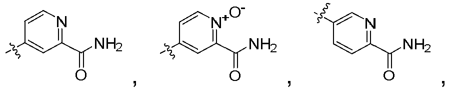 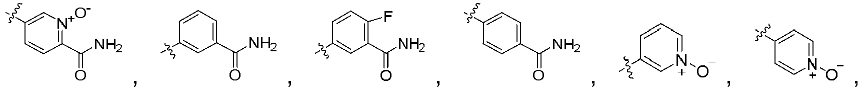 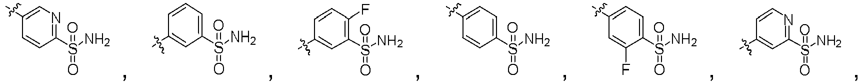 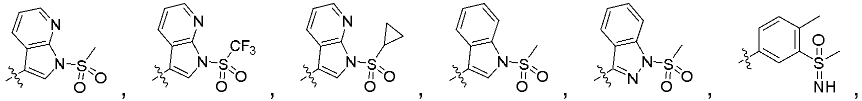 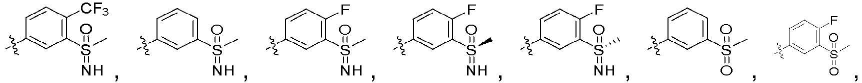 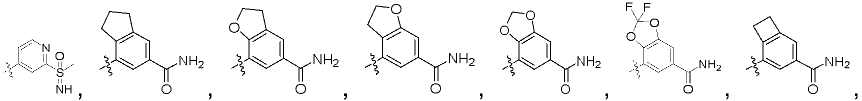 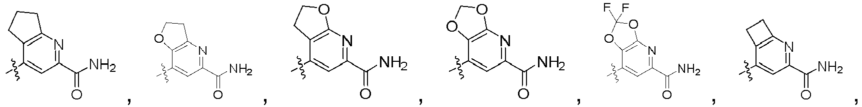 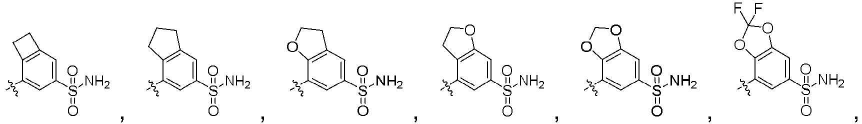 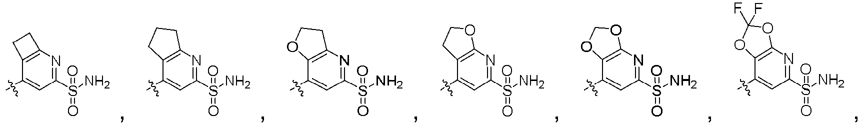 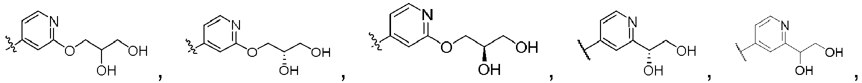 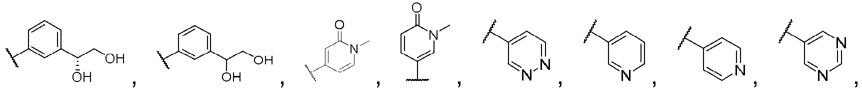 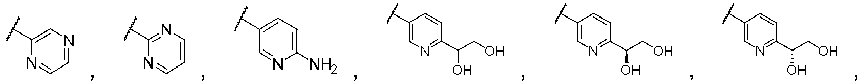 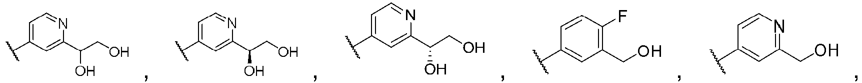 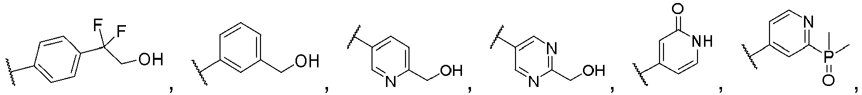 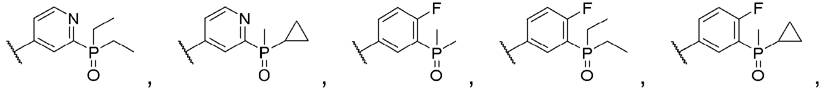 or 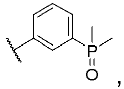 Q₁ being optionally substituted with 1 to 3 R^{q}'s; and
B is selected from 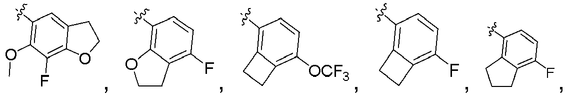 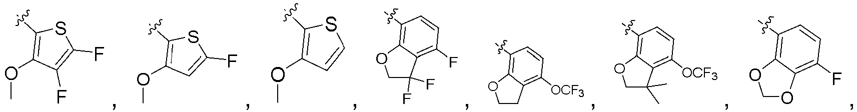 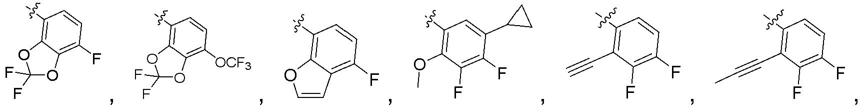 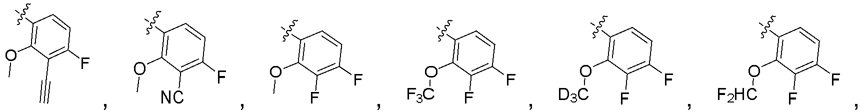 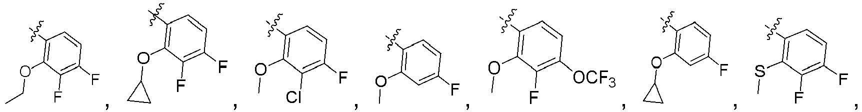 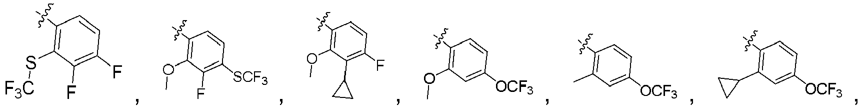 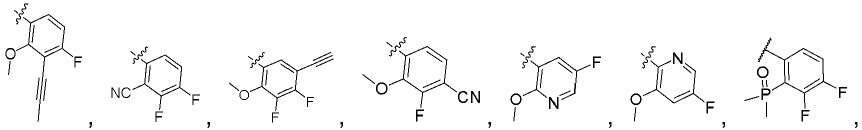 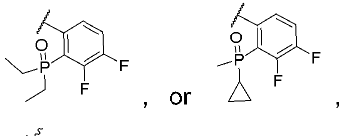 preferably

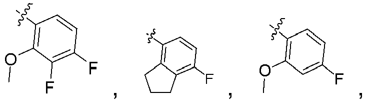

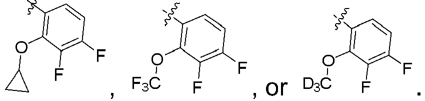

7. The compound or a stereoisomer, a deuterate, a solvate, a prodrug, a metabolite, a pharmaceutically acceptable salt, or a cocrystal thereof according to claim 1, wherein the compound is selected from one of the structures shown in Table E.

8. A pharmaceutical composition, comprising the compound or a stereoisomer, a deuterate, a solvate, a prodrug, a metabolite, a pharmaceutically acceptable salt, or a cocrystal thereof according to any one of claims 1 to 7, and a pharmaceutically acceptable carrier; preferably, the pharmaceutical composition comprising 1 mg to 1500mg of the compound or a stereoisomer, a deuterate, a solvate, a prodrug, a metabolite, a pharmaceutically acceptable salt, or a cocrystal thereof according to any one of claims 1 to 7.

9. Use of the compound or a stereoisomer, a deuterate, a solvate, a prodrug, a metabolite, a pharmaceutically acceptable salt, or a cocrystal thereof according to any one of claims 1 to 7 or the pharmaceutical composition according to claim 8 in manufacturing a medicament for treating or/and alleviating pain.

10. A method for treating or alleviating a disease in a mammal, the method comprising administering to a subject a therapeutically effective amount of the compound or a stereoisomer, a deuterate, a solvate, a prodrug, a metabolite, a pharmaceutically acceptable salt, or a cocrystal thereof according to any one of claims 1 to 7 or the pharmaceutical composition according to claim 8, the therapeutically effective amount being preferably 1 mg to 1500 mg, and the disease being preferably pain.
